# EUROPEAN PATENT APPLICATION

(11) **EP 3 520 604 A1**
(43) Date of publication of application: **07.08.2019**
(21) Application number: 17855825.0
(22) Date of filing: 19.09.2017
(51) Int. Cl.: A01H 5/00, A01G 7/06, A01G 31/00

(54) **PLANT BODY, PRODUCTION METHOD FOR PLANT BODY, RESISTANCE IMPARTING METHOD, TOMATO AND PRODUCTION METHOD FOR TOMATO**

(30) Priority: 29.09.2016 JP 2016191962; 29.09.2016 JP 2016191983; 29.09.2016 JP 2016192096
(71) Applicant: Sekisui Chemical Co., Ltd., Osaka-shi, Osaka 530-8565 (JP)
(72) Inventor: SAKAKIBARA, Motohiro, Tsukuba-shi, Ibaraki 300-4292 (JP); TOGI, Akiko, Tsukuba-shi, Ibaraki 300-4292 (JP); ONO, Seigo, Tsukuba-shi, Ibaraki 300-4292 (JP); NISHIGUCHI, Naoki, Tsukuba-shi, Ibaraki 300-4292 (JP); GOTOU, Hiroaki, Tsukuba-shi, Ibaraki 300-4292 (JP)
(74) Representative: Ter Meer Steinmeister & Partner
(86) International application number: PCT/JP2017/033726
(87) International publication number: WO 2018/061870

(57) **Abstract**

A plant corresponding to an item described in Standard Tables of Food Composition in Japan 2015 (Seventh Revised Version) and having a mass content of sodium which is at least 50 times a mass content of sodium of the item described in the Standard Tables of Food Composition in Japan 2015 (Seventh Revised Version), the mass content of sodium being measured per unit mass of edible portion of the plant.

## Description

### TECHNICAL FIELD

The present invention relates to a sodium-rich plant, a method for producing the plant, a method for imparting tolerance to a plant against diseases and pests, a tomato, and a method for producing a tomato.

Priorities are claimed on Japanese Patent Application Nos. 2016-192096, 2016-191983 and 2016-191962, each filed September 29, 2016, the contents of which are incorporated herein by reference.

### BACKGROUND ART

For improving the shelf life of crops, it has been widely practiced to treat plants with preservatives. As preservatives, for example, sodium chlorite aqueous solution, fumarate, acetic acid, alum and the like are known (see, for example, Patent Documents 1 to 4). However, the excessive use of preservatives raises concern about their influence on health, etc., and suppression of the use of preservatives has been desired.

Spread of diseases and pests (harmful insects) that may harm the plant growth leads to decrease in the yield of crops, causing large economic losses. For preventing crops from suffering from diseases and pests, agricultural chemicals have been widely used in growing plants. However, the excessive use of agricultural chemicals raises concern about their influence on ecosystem, or safety of the chemicals per se, so that suppression of the use of agricultural chemicals has been desired.

As a method for imparting tolerance to plants against diseases and pests, a method using gene manipulation is known (see, for example, Patent Document 5). However, genetically modified plants have a problem related to safety concerns; therefore, it is desirable to impart tolerance to plants without using gene manipulation.

Tomatoes are widely distributed to be eaten raw or processed. In fact, tomatoes are often eaten raw and those having high nutritional value as well as good taste and flavor are favored.

In recent years, tomatoes known as "fruit tomatoes" which have a higher sugar content than conventional tomatoes are gaining popularity. In addition, tomatoes cultivated in soil with high salt concentration in Kumamoto prefecture are called "salt tomatoes", and sold as high-grade tomatoes with high sugar content. However, the yield of salt tomatoes that can be harvested is limited, and it is reported that the yield tends to be very unstable (see, for example, Non-Patent Document 1).

### Prior Art References

### Patent Document

Patent Document 1: Japanese Unexamined Patent Application, First Publication No. 2005-130691
Patent Document 2: Japanese Unexamined Patent Application, First Publication No. 2000-342170
Patent Document 3: Japanese Unexamined Patent Application, First Publication No. Hei 9-140365
Patent Document 4: Japanese Unexamined Patent Application, First Publication No. Hei 11-137171
Patent Document 5: Japanese Unexamined Patent Application, First Publication No. 2014-076052

### Non-Patent Document

Non-Patent Document 1: Morita, "The Trend of Production in a Tomato Production Center and Technical Development Needs of the Producers", Proceedings of vegetable and tea science, National Institute of Vegetable and Tea Science, National Agriculture and Bio-oriented Research Organization, March 2006, No. 3, p.85 - 90

### SUMMARY OF THE INVENTION

### Problems to be Solved by the Invention

In view of the problems described above, an object of the present invention is to provide a plant with improved shelf life as compared to the conventional plant of the same species, and a method for producing the plant.

In view of the problems described above, another object of the present invention is to provide a method for imparting excellent tolerance to a plant against diseases and pests.

In view of the problems described above, still another object of the present invention is to provide a tomato which can be stably harvested and has improved flavor, and a method for producing the tomato.

### Means to Solve the Problems

The plant and the method for producing the plant according to the present invention are as described below in [1] through [6].
[1] A plant corresponding to an item described in Standard Tables of Food Composition in Japan 2015 (Seventh Revised Version) and having a mass content of sodium which is at least 50 times a mass content of sodium of the item described in the Standard Tables of Food Composition in Japan 2015 (Seventh Revised Version), the mass content of sodium being measured per unit mass of edible portion of the plant.
[2] The plant according to [1], which has a sodium content of 0.15 % by mass or more as measured with respect to edible portion of a fruit of the plant.
[3] The plant according to [1] or [2], which has a water content of 90 % by mass or less as measured with respect to edible portion of a fruit of the plant.
[4] The plant according to any one of [1] to [3], which has a sugar content (Brix) of 8 or more as measured with respect to edible portion of a fruit of the plant.
[5] The plant according to any one of claims [1] to [4], which is a tomato.
[6] A method for producing the plant of any one of [1] to [5], including:
   a salt tolerance imparting step of performing a salt resistance imparting treatment by bringing a salt tolerance imparting agent into contact with at least a part of a root of a plant, thereby obtaining a salt tolerance imparted plant; and
   a cultivation step of hydroponically cultivating the salt tolerance imparted plant obtained in the salt tolerance imparting step with a nutrient solution having a sodium chloride concentration of 1 % by mass or more.

The tolerance imparting method according to the present invention is as described below in [1] through [10].
[1] A tolerance imparting method for imparting tolerance to a plant against diseases and pests, including:
   a salt tolerance imparting step of performing a salt resistance imparting treatment by bringing a salt tolerance imparting agent into contact with at least a part of a root of a plant, thereby obtaining a salt tolerance imparted plant; and
   a cultivation step of hydroponically cultivating the salt tolerance imparted plant obtained in the salt tolerance imparting step with a nutrient solution having a sodium chloride concentration of 1 % by mass or more.
[2] The tolerance imparting method according to [1], which further includes, as a step to be performed prior to the salt tolerance imparting step, an initial growth step of allowing a seed or a bulb of the plant to bud and root under an environment with a sodium chloride concentration of less than 1 % by mass.
[3] The tolerance imparting method according to [2], wherein the seed or the bulb is allowed to bud and root in the initial growth step under an environment with a sodium chloride concentration of 0.5 % by mass or less.
[4] The tolerance imparting method according to any one of [1] to [3], wherein the salt tolerance imparting treatment is a treatment of putting at least a part of the root of the plant into a treatment solution which contains the salt tolerance imparting agent, and has a sodium chloride concentration of 1 % by mass or more.
[5] The tolerance imparting method according to [4], wherein at least a part of the root of the plant is held in the treatment solution for 1 hour or more.
[6] The tolerance imparting method according to [4] or [5], wherein the salt tolerance imparting agent is a microorganism that imparts the salt tolerance to the plant by adhering to the root, and
   the concentration of the microorganism in the treatment solution is 10³ CFU/mL or more.
[7] The tolerance imparting method according to any one of [1] to [6], wherein the nutrient solution further contains a magnesium chloride in an amount of 0.5 % by mass or less.
[8] The tolerance imparting method according to any one of [1] to [7], wherein the salt tolerance imparting agent comprises at least one species of the microorganism.
[9] The tolerance imparting method according to any one of [1] to [8], wherein the plant is a plant of Solanaceae family.
[10] The tolerance imparting method according to any one of [1] to [9], wherein the tolerance is viral disease tolerance.
[11] The tolerance imparting method according to any one of [1] to [9], wherein the tolerance is fungal disease tolerance.

The tomato and the method for producing the tomato according to the present invention are as described below in [1] through [7].
[1] A tomato satisfying at least one of the following (1) to (20), which respectively represent amounts of free amino acids contained per 100 g of edible portion of a fruit of the tomato:
   (1) free glutamic acid : 200 mg or more,
   (2) free aspartic acid : 40 mg or more,
   (3) free arginine : 6 mg or more,
   (4) free isoleucine : 6 mg or more,
   (5) free alanine : 8 mg or more,
   (6) free serine : 15 mg or more,
   (7) free lysine : 7 mg or more,
   (8) free histidine : 7 mg or more,
   (9) free phenylalanine : 12 mg or more,
   (10) free tyrosine : 4 mg or more,
   (11) free leucine : 4 mg or more,
   (12) free methionine : 2 mg or more,
   (13) free valine : 3.5 mg or more,
   (14) free glycine : 2 mg or more,
   (15) free proline : 50 mg or less,
   (16) free threonine : 10 mg or more,
   (17) free tryptophan : 2 mg or more,
   (18) free phosphoserine : 1.2 mg or more,
   (19) free β-alanine : 2 mg or more, and
   (20) free γ-aminobutyric acid : 80 mg or more.
[2] The tomato according to [1], which satisfies at least one of (1), (2), (4) to (6), (8), (13), (14) and (16) .
[3] The tomato according to [2], which satisfies at least one of (1), (5), (6), (14) and (16).
[4] The tomato according to [1], which satisfies at least one of (1) to (6), wherein the amounts of free amino acids contained per 100 g of edible portion of a fruit of the tomato are as follows:
   (1) free glutamic acid : 500 mg or more,
   (2) free aspartic acid : 100 mg or more,
   (3) free arginine : 10 mg or more,
   (4) free isoleucine : 10 mg or more,
   (5) free alanine : 10 mg or more, and
   (6) free serine : 70 mg or more.
[5] The tomato according to [4], which satisfies at least one of (1) and (2):
   (1) free glutamic acid : 500 mg or more, and
   (2) free aspartic acid : 100 mg or more.
[6] The tomato according to any one of [1] to [5], which has a sodium content of 0.15 % by mass or more as measured with respect to edible portion of a fruit of the tomato.
[7] The tomato according to any one of [1] to [6], which has a water content of 90 % by mass or less as measured with respect to edible portion of a fruit of the tomato.
[8] The tomato according to any one of [1] to [7], which has a sugar content (Brix) of 8 or more as measured with respect to edible portion of a fruit of the tomato.
[9] A method for producing the tomato of any one of [1] to [7], including:
   a salt tolerance imparting step of performing a salt resistance imparting treatment by bringing a salt tolerance imparting agent into contact with at least a part of a root of a tomato, thereby obtaining a salt tolerance imparted tomato; and
   a cultivation step of hydroponically cultivating the salt tolerance imparted tomato obtained in the salt tolerance imparting step with a nutrient solution having a sodium chloride concentration of 1 % by mass or more.

### Effect of the Invention

The present invention can provide a plant with improved shelf life as compared to the conventional plant of the same species.

The tolerance imparting method of the present invention can impart tolerance to plants against diseases and pests.

A fruit of the tomato of the present invention has free amino acid contents different from those of conventional tomatoes and has unique flavor.

The tomato production method of the present invention can provide a tomato bearing a fruit having free amino acid contents different from those of conventional tomatoes and unique flavor.

### BRIEF DESCRIPTION OF THE DRAWINGS

FIG. 1A is a photograph of tomatoes grown by hydroponics in Comparative Example 1B.
FIG. 1B is a photograph of tomatoes grown by hydroponics in Example 1B.
FIG. 2 is a photograph of tomatoes grown by hydroponics in Example 2B.
FIG. 3 shows photographs of tomatoes grown by hydroponics in Example 3B and Comparative Example 2B.

### DESCRIPTION OF THE EMBODIMENTS

### ≪Plant≫

The plant of the present invention corresponds to an item described in Standard Tables of Food Composition in Japan 2015 (Seventh Revised Version) and has a mass content of sodium which is at least 50 times a mass content of sodium of the item described in the Standard Tables of Food Composition in Japan 2015 (Seventh Revised Version), the mass content of sodium being measured per unit mass of edible portion of the plant.

The plant of the present invention corresponds to an item described in Standard Tables of Food Composition in Japan 2015 (Seventh Revised Version) and has a mass content of sodium which is preferably 55 to 100 times, more preferably 60 to 90 times, still more preferably 70 to 80 times a mass content of sodium of the item described in the Standard Tables of Food Composition in Japan 2015 (Seventh Revised Version), the mass content of sodium being measured per unit mass of edible portion of the plant.

The plant of the present invention preferably has a sodium content of 0.15 % by mass or more as measured with respect to edible portion of a fruit of the plant. The sodium content is more preferably 0.18 to 0.4 % by mass, and still more preferably 0.2 to 0.3 % by mass. The sodium content can be measured by known method.

The plant of the present invention is a raw plant which is not processed except cutting and is preferably a fruit.

When the plant of the present invention is a fruit, the plant preferably has a water content of 90 % by mass or less as measured with respect to edible portion of a fruit of the plant. The water content is more preferably 80 to 90 % by mass, still more preferably 83 to 89 % by mass, and still more preferably 85 to 88% by mass. The water content can be measured by known method. For example, the water content can be determined by a heat drying method using a commercially available dryer.

When the plant of the present invention is a fruit, the plant preferably has a sugar content (Brix) of 8 or more as measured with respect to edible portion of a fruit of the plant. The sugar content (Brix) is more preferably 8 to 20, still more preferably 10 to 15, and still more preferably 12 to 14. The sugar content can be measured by known method. For example, the sugar content can be measured using a commercially available sugar content refractometer.

The edible part of the fruit means a part of the harvested fruit excluding parts such as calyx, stem and possibly seeds. The fruit is an unprocessed raw fruit.

With respect to the fruit of the present invention, the sodium content, the water content, and the sugar content can be determined by measuring the respective amounts with respect to the whole edible part of the fruit.

The plant of the present invention may be an angiosperm, a gymnosperm, or ferns or moss.

Further, the plant may be a monocotyledonous plant or a dicotyledonous plant. Specific examples of the plant include plants of the Gramineae family such as rice, corn, sorghum, wheat, barley, rye, barnyard millet, or foxtail millet; the plant of the Solanaceae family such as tomato, eggplant, paprika, bell pepper, potato, or tobacco; plants of the Brassicaceae family such as Arabidopsis thaliana, colza, shepherd's purse, radish, cabbage, violet cabbage, Brussels sprouts (Petit vert), Chinese cabbage, bok choy, kale, watercress, Japanese mustard spinach, broccoli, cauliflower, turnip, Japanese horseradish, or mustard; plants of the Cucurbitaceae family such as cucumber, bitter melon, pumpkin, melon, or watermelon; plants of the Vitaceae family such as grape; plants of the Rutaceae family such as lemon, orange, navel orange, grapefruit, mandarin orange, lime, Citrus sudachi, citron, Shikuwasa, or citrus tankan; plants of the Rosaceae family such as apple, cherry, Japanese apricot, peach, loquat, apricot, plum (Prumus salicina), prune, almond, pear, European pear, strawberry, raspberry, blackberry, black currant, cranberry, or blueberry; plants of the Leguminosae family such as soybean, kidney bean, pea, broad bean, green soybean, green gram, or chick pea; plants of the Nelumbonaceae family such as lotus (lotus root); plants of the Pedaliaceae family such as sesame; plants of the Chenopodiaceae family such as spinach, beet, sugar beet, quinoa, tumbleweed, amaranth, or cockscomb; plants of the Arecaceae family such as date palm, oil palm, coconut, or acai; plants of the Musaceae family such as banana, Musa basjoo, or Manila hemp; plants of the Malvaceae family such as cotton or okra; plants of the Myrtaceae family such as eucalyptus; and plants of the Capparaceae family such as Cleome gynandra or Cleome spinose.

Among these plants, the plants of the Solanaceae family are preferable, and tomato (Solanum lycopersicum) is more preferable.

The degree of ripening of tomato can be classified by the extent (area%) of red or pink coloration on the fruit surface. For example, a tomato goes through a green ripe period (no coloration), a degreening period (up to 70 % coloration), a mature period (71 to 90 % coloration), and a ripe period (91 to 100 % coloration) to reach an over-ripe period. With respect to the fruit of the present invention, it is preferable that the sodium content, the water content and the sugar content are within the aforementioned ranges in the mature period or the ripe period.

The plant of the present invention has unique flavor due to the aforementioned specific sodium content.

Further, the sodium content per mass of the plant is high, whereby the plant can maintain good flavor for a long period of time and has a long shelf life.

The plant of the present invention can exhibit excellent shelf life even without treating the harvested plant with a preservative.

### ≪Plant Production Method≫

The plant production method of the present invention includes: a salt tolerance imparting step of performing a salt resistance imparting treatment by bringing a salt tolerance imparting agent into contact with at least a part of a root of a plant, thereby obtaining a salt tolerance imparted plant; and a cultivation step of hydroponically cultivating the salt tolerance imparted plant obtained in the salt tolerance imparting step with a nutrient solution having a sodium chloride concentration of 1 % by mass or more. The salt tolerance imparting step is carried out for imparting salt tolerance to a plant originally having low salt tolerance by treating the plant with the salt tolerance imparting agent, thereby enabling the plant to be cultivated under an environment of very high salt concentration such as the sodium chloride concentration of 1 % by mass or more. In the subsequent cultivation step, the salt tolerance imparted plant can be grown by hydroponics with the nutrient solution having a sodium chloride concentration of 1 % by mass or more.

By performing the salt tolerance imparting step and the cultivation step, sodium derived from sodium chloride contained in the nutrient solution is transferred into the plant, whereby the plant of the present invention containing a large amount of sodium can be produced.

A plant at an early stage of growth has less stress tolerance than a sufficiently grown plant, and is more likely to be influenced by environmental stress. In particular, the process of rooting or budding is very sensitive to the salt concentration. Therefore, when grown under a high salt concentration environment from the stage of seeds or bulbs, many of the plants are caused to wither due to high salt stress before acquiring sufficient salt tolerance even if the salt tolerance imparting treatment is carried out. In the plant production method of the present invention, it is preferable that the plant is grown under a low salt concentration environment at an early stage of the growth, and the salt tolerance imparting treatment is performed after the plant has been grown to a certain extent. This can noticeably increase a proportion of the plants to which the salt tolerance is imparted by the salt tolerance imparting treatment, thereby making it possible to efficiently raise seedlings capable of being cultivated under a high salt concentration environment.

The plant production method of the present invention preferably includes, as an initial growth step, a step of allowing the plant to grow under an environment with a sodium chloride concentration of less than 1 % by mass, at least until the rooting and the budding are completed. The sodium chloride concentration of the environment in which the seed or the like is grown in the initial growth step may be less than 1 % by mass, and is preferably equal to or less than the salt concentration at which a plant of the same variety as the plant to be obtained by raising the seedling is capable of being normally grown. The environment that allows "capable of being normally grown" means an environment in which the cultivation of a plurality of plants results in a survival rate of 80 % or more. In the tolerance imparting method of the present invention, the sodium chloride concentration of the environment for the initial growth step is preferably 0 to 0.5 % by mass, more preferably 0 to 0.3 % by mass, and further preferably 0 to 0.1 % by mass.

The initial growth step can be performed by a general method for causing the seed to bud and root, except that an aqueous solution having a sodium chloride concentration of less than 1 % by mass is used as water (initial growth solution) to be supplied to the seed or the bulb. Specifically, the seed or the bulb is allowed to bud and root by placing the seed or the bulb in a state of being in contact with the initial growth solution under a temperature environment which allows budding and rooting. For example, the initial growth solution may be periodically sprayed onto the seed or the like which is placed under a suitable temperature environment. Alternatively, the seed or the like may be placed in a state where at least a part of the surface of the seed or the like is exposed to air while other parts are in contact with the initial growth solution under a suitable temperature environment. For example, the seed or the like may be placed on the surface of a support including the initial growth solution, thereby allowing the seed or the like to be in contact partially with the initial growth solution. Alternatively, the seed or the like is placed in the initial growth solution that is held in a container such that the surface of the solution is lower than the top of the seed or the like, to thereby allow the seed or the like to be in contact partially with the initial growth solution.

The support is not limited as long as the support has such a porosity that allows the initial growth solution contained therein to be supplied to the seed or the like placed on the surface of the carrier. However, it is preferable that the support has such a porosity that, after rooting, allows the root of the seedling to penetrate through the support. By allowing the plant budded and rooted from the seed or the like to grow such that a stem or a leaf grows upward from the support, and the root grows downward into the support, the plant can grow in a state of being supported by the support. For example, when the plant is grown such that the seed or the like is allowed to bud and root by placing the seed or the like on the surface of the support retained within a cultivation pot installable in a cultivation tank used for the hydroponics performed in the cultivation step, while allowing the root to grow downward into the support so as to penetrate through the support, it is possible, even after the seedling stage, to grow the plant by installing the cultivation pot as such in the cultivation tank, since the plant is supported in a state of being retained in the cultivation pot.

As a support having such porosity, for example, a gel material, a fiber-shaped material, or a granular or gravel-shaped material may be used. Examples of the gel material include polysaccharide polymers such as agar, agarose, gellan gum, and alginic acid; and water absorptive resins such as acrylic resin. Examples of the fiber-shaped material include non-woven fabric, cotton, paper, rock wool, and glass wool. Examples of the granular or gravel-shaped material include a wood chip, a bark, pumice, vermiculite, and sand.

After the initial growth step, the salt tolerance imparting treatment may be performed, as a salt tolerance imparting step, by bringing the salt tolerance imparting agent into contact with at least a part of a root of the grown seedling. The salt tolerance imparting treatment may be performed immediately after budding and rooting, but this treatment can enhance the tolerance against salt stress more as the seedling grows more. Therefore, it is preferable that the salt tolerance imparting step is performed after the seedling is grown for at least one week, and preferably for approximately three weeks, after budding.

The salt tolerance imparting treatment can be performed by putting at least a part of the root of the seedling into an aqueous solution (treatment solution) containing the salt tolerance imparting agent. The sodium chloride concentration of the treatment solution is not particularly limited, and may be appropriately adjusted depending on a kind of the salt tolerance imparting agent used or a kind of the plant such that a sufficient salt tolerance imparting efficiency is obtained. For example, the treatment solution may be a solution obtained by mixing the salt tolerance imparting agent with the initial growth solution, a solution obtained by mixing the salt tolerance imparting agent with the nutrient solution used in the cultivation step, or a solution having a salt composition different from those of the initial growth solution and the nutrient solution. The sodium chloride concentration of the treatment solution used in the present invention is preferably 1 % by mass or more, and more preferably the same as the sodium chloride concentration of the nutrient solution.

The salt tolerance imparting agent used in the present invention may be a drug, a microorganism, or a culture supernatant of the microorganism. Examples of the drug include pyrroloquinoline quinone (see Japanese Patent No. 5013326) and strigolactone. Examples of the microorganism include Paenibacillus Fukuinensis (see Japanese Unexamined Patent Application, First Publication No. 2013-75881). The salt tolerance imparting agent may be formed of one kind of microorganism, or a mixture of two or more kinds of microorganisms.

The concentration of the salt tolerance imparting agent in the treatment solution can be appropriately adjusted in consideration of the kind of the salt tolerance imparting agent, the kind of the plant, the growth stage or the like. When the concentration of the salt tolerance imparting agent in the treatment solution is too low, the salt tolerance imparting agent is less likely to get in contact with the root of the plant in the treatment solution, which may result in insufficient salt tolerance imparting effect. On the other hand, depending on the kind of the salt tolerance imparting agent, the growth of the plant may be adversely affected by excessive intake of the salt tolerance imparting agent. In view of this, an appropriate concentration of the salt tolerance imparting agent in the treatment solution for obtaining sufficient salt tolerance imparting effect can be determined empirically. For example, when the salt tolerance imparting agent is the microorganism, the concentration of the microorganism in the treatment solution may be set to be 10³ CFU/mL or more, whereby sufficient salt tolerance imparting effect can be obtained. When the salt tolerance imparting agent is the microorganism, the concentration of the microorganism in the treatment solution is preferably 10⁴ CFU/mL or more, and more preferably 10⁵ CFU/mL or more. When the salt tolerance imparting agent is the microorganism, an upper limit of the concentration of the microorganism in the treatment solution is not particularly limited, but for example, the concentration may be up to 10¹³ CFU/mL, whereby the quality of the treatment solution can be favorably maintained. The upper limit of the concentration of the microorganism in the treatment solution is preferably 10¹² CFU/mL, more preferably 10¹¹ CFU/mL, still more preferably 10¹⁰ CFU/mL, particularly preferably 10⁹ CFU/mL. The range of the concentration of the microorganism in the treatment solution may be, for example, 10³ to 10¹³ CFU/mL, preferably 10⁴ to 10¹² CFU/mL, more preferably 10⁵ to 10¹¹ CFU/mL, still more preferably 10⁵ to 10¹⁰ CFU/mL, especially preferably 10⁵ to 10⁹ CFU/mL.

The time period for performing the salt tolerance imparting treatment time once, that is, the time for holding at least a part of the root of the plant in the treatment solution, can be appropriately adjusted in consideration of the kind of the plant or the kind of the salt tolerance imparting agent to be used. For example, such time period for the salt tolerance imparting treatment is preferably 1 hour or more, more preferably 18 hours or more, still preferably one day or more, and further more preferably one day to seven days. Cultivating the plant for 1 hour or more with the root thereof being held in the treatment solution ensures sufficient chances for the salt tolerance imparting agent in the treatment solution to contact the root of the plant, whereby the salt tolerance can be imparted more easily.

In the initial growth step, when the seedling is to be grown in a state of being supported by the support retained within the cultivation pot, the cultivation pot may be installed in a treatment tank containing the treatment solution such that the root growing from below the support contacts the treatment solution, thereby carrying out the salt tolerance imparting treatment. For example, the root can be brought into contact with the treatment solution by using a float which has one or more through-holes for fitting the cultivation pot thereinto and is floated on the surface of the treatment solution, and fitting the cultivation pot into the float. The cultivation pot may be detachably fitted into the through-hole of the float, or may be fixed undetachably to the through-hole of the float. Alternatively, the float and the cultivation pot may be integrally formed. As a material of the float to be floated on the surface of the treatment solution, the same material as the float to be floated on the surface of the nutrient solution described later can be used.

The larger the amount of the treatment solution used in the salt tolerance imparting treatment, the larger the amount of the salt tolerance imparting agent needed. Therefore, by reducing the amount of the treatment solution to an amount that is necessary and sufficient to allow the root of the plant grown from the bottom surface of the cultivation pot to contact the treatment solution, the amount of the salt tolerance imparting agent required for performing the salt tolerance imparting treatment once can be suppressed. However, when the amount of the treatment solution is too small, it may become impossible to allow a sufficient amount of the salt tolerance imparting agent to contact the root of the plant. Therefore, when a plate into which one cultivation pot is fitted is installed per treatment tank, the amount of the treatment solution contained in the treatment tank is preferably at least 5 mL.

Thereafter, the seedling to which the salt tolerance is imparted by the salt tolerance imparting step is grown by hydroponics with the nutrient solution having a sodium chloride concentration of 1 % by mass or more.

In the present invention, the sodium chloride concentration of the nutrient solution in the cultivation step is not limited as long as the sodium chloride concentration is 1 % by mass or more, and may be appropriately adjusted in accordance with the salt tolerance of the plant to be cultivated. The sodium chloride concentration of the nutrient solution is preferably 1 to 4 % by mass, more preferably 1.5 to 3.8 % by mass, further preferably 2 to 3.5 % by mass, and particularly preferably 2.5 to 3.3 % by mass.

The nutrient solution used in the present invention preferably contains magnesium chloride in addition to sodium chloride, where the amount of magnesium chloride contained is preferably 0.5 % by mass or less, more preferably 0.1 to 0.5 % by mass.

In addition to sodium chloride and magnesium chloride, it is preferable that the nutrient solution used in the present invention contains various nutrient components which are necessary for the growth of the plant. The nutrient components can be appropriately adjusted according to the type of the plant to be cultivated. Especially, it is preferable that the nutrient solution contains elements necessary for the growth of the plant in the form of salts. Examples of such elements include nitrogen, phosphorus, potassium, calcium, magnesium, sulfur, iron, manganese, copper, molybdenum, and boron. The nutrient solution may further contain elements such as aluminum and silicon in the form of salts thereof, depending on the type of the plant. Further, the composition of the nutrient solution may be varied according to the growth stage of the plant.

The nutrient solution to be used in the present invention may be, for example, a solution prepared by supplementing deficient salt such as sodium chloride to commercially available liquid fertilizer or a solution obtained by diluting commercially available concentrated liquid fertilizer with sea water instead of water. Further, the nutrient solution may also be a solution obtained by appropriately adding a deficient salt such as salt of phosphorus to seawater.

In the present invention, the hydroponics in the cultivation step may be performed by a general hydroponics method, except that the sodium chloride concentration of the nutrient solution is set to 1 % by mass or more. The cultivation step may be performed by a deep flow technique in which a relatively large amount of the nutrient solution is stored in the cultivation tank, or a nutrient film technique in which a culture solution is allowed to flow down little by little on a flat surface having a gentle slope.

In the deep flow technique, the replacement of the nutrient solution in the cultivation tank may be carried out by a circulation method in which the nutrient solution used is circulated, or a non-circulation method in which the nutrient solution used for a certain period of time in the cultivation tank is discharged. In case of the circulation method, the nutrient solution prepared in a nutrient solution preparation tank is charged into the cultivation tank by a pump or the like, and is collected back to the nutrient solution preparation tank from the cultivation tank, and the nutrient component or the like is prepared.

For example, the deep flow technique can be carried out by using a hydroponic apparatus having a cultivation tank for storing the nutrient solution, a cultivation pot for accommodating the plant, one or more through-holes for fitting the cultivation pot, and a float to be floated on the surface of the nutrient solution. The cultivation pot may be detachably fitted into the through-hole of the float, or may be fixed undetachably to the through-hole of the float. Alternatively, the float and the cultivation pot may be integrally formed. The cultivation tank may be installed indoors, or may be installed outdoors.

In the case of a circulation type hydroponic apparatus, the cultivation tank includes a water supply hole for injecting the nutrient solution, and a drainage hole for draining the nutrient solution. In the case of a non-circulation type hydroponic apparatus, the cultivation tank may include both of the water supply hole and the drainage hole, or may include a water supply/drainage hole used for both of the water supply and the drainage. The water supply and drainage of the nutrient solution in the cultivation tank are controlled by a pump and a valve.

The cultivation pot is a container that has opening portions at least on an upper surface and a lower surface, and is capable of retaining the support. In general, the cultivation pot formed of a resin material such as polyethylene, polypropylene, or polyvinylidene chloride is used. As the support to be retained in the cultivation pot, any of those described above can be used.

The float is formed of a material that floats on the surface of the nutrient solution in a state where the cultivation pot being used for cultivating the plant is fitted into the through-hole. As such a material, for example, a foamed resin such as polystyrene foam or polypropylene foam is used. By fitting the cultivation pot into the float, the cultivation pot can be constantly positioned on the surface of the nutrient solution, regardless of a large or small amount of the nutrient solution, and even if the amount of the nutrient solution is small, the root of the plant can be allowed to contact constantly with the nutrient solution.

The float to be floated in the cultivation tank may be one sheet, or two or more sheets. When the cultivation tank is installed outdoors, it is preferable that the float is installed to cover most of the surface of the nutrient solution, in order to prevent evaporation of the nutrient solution from the surface thereof.

In the deep flow technique, it is preferable that the hydroponic apparatus used includes oxygen supply means for keeping the dissolved oxygen content of the nutrient solution at a predetermined level or higher. As the oxygen supply means, for example, an air pump or an air sucker can be used. By installing the air pump in the cultivation tank, air including oxygen can be directly supplied to the nutrient solution in the cultivation tank. When the air sucker is used, the nutrient solution can be charged into the cultivation tank after the nutrient solution is mixed with air by passing the nutrient solution through the air sucker or the like in advance.

The pH suitable for the hydroponics varies depending on the kind of the plant, and is generally approximately 5.5 to 6.5, but the pH of the nutrient solution tends to increase as the cultivation period extends longer. Therefore, in order to stably perform the hydroponics for a long period of time, it is preferable that the hydroponic apparatus used includes pH control means for measuring the pH of the nutrient solution periodically, and dosing an acid material in order to adjust the pH within a predetermined range as necessary. As the acid material used for the pH adjustment, for example, hydrochloric acid, sulfuric acid, or nitric acid can be used.

In the plant production method of the present invention, the salt tolerance imparting step and the cultivation step may be performed in the same cultivation tank, or the salt tolerance imparting step may performed in the treatment tank storing the treatment solution, followed by transferring the seedling after the treatment to the cultivation tank storing the nutrient solution.

In the initial growth step, when the salt tolerance imparting treatment is performed in the treatment tank after the seedling is grown in a state of being supported by the support retained within the cultivation pot, the cultivation pot with the seedling may be detached from the float of the treatment tank, and may be fitted into the through-hole of the float floating on the surface of the nutrient solution stored in the cultivation tank, or the float into which the cultivation pot is embedded may be transferred from the treatment tank to be floated on the surface of the nutrient solution in the cultivation tank. Transfer means for transferring the cultivation pot or the float to the cultivation tank from the treatment tank is not particularly limited, and for example, the transfer may be performed by means of a water current, or a conveyor. When a plurality of cultivation pots are installed per treatment tank, it is preferable that a bubbling treatment is performed by the air pump, in order to prevent stagnation of the treatment solution, and oxygen deficiency.

When the salt tolerance imparting treatment is performed in the cultivation tank, first, the treatment solution is charged to the cultivation tank, and the root grown downward in the cultivation pot fitted into the float is brought into contact with the treatment solution to thereby perform the salt tolerance imparting treatment. In order to prevent the occurrence of concentration gradient of the salt tolerance imparting agent, it is preferable that the treatment solution is contacted with the root of the plant while suppressing the amount of the water supply and drainage or without the water supply and drainage.

However, when the amount of the water supply and drainage is small or the water supply and drainage is not performed, the stagnation may occur in the cultivation tank, causing adverse effect on the plant itself. Therefore, it is preferable that the treatment solution is suitably stirred by the bubbling treatment with the air pump.

After the salt tolerance imparting treatment, the treatment solution in the cultivation tank is drained, and subsequently, the nutrient solution prepared in advance in another tank is supplied to the cultivation tank. Then, the cultivation step is initiated by supplying water and draining under normal conditions. When the salt tolerance imparting agent is composed of a material, such as the microorganism, which does not adversely affect the plant even in the event of excessive intake of the material, the nutrient solution may be supplied without draining the treatment solution, and the water supply and drainage may be initiated under the normal water supply and drainage conditions.

The cultivation with the nutrient solution having a sodium chloride concentration of 1 % by mass or more (hereinafter, also referred to as "under the high salt concentration environment") need not necessarily be performed throughout the entire cultivation period after the salt tolerance imparting treatment. For example, the cultivation under the high salt concentration environment may be performed only for an arbitrary period after the salt tolerance imparting treatment. In this case, it is preferable that the cultivation under the high salt concentration environment is performed for a predetermined period immediately after the salt tolerance imparting treatment. It is speculated that, by performing the cultivation under the high salt concentration environment at an arbitrary time immediately after the salt tolerance imparting treatment, the salt tolerance imparted by the salt tolerance imparting treatment is maintained, whereby the shelf life of the plant improves. The period of the cultivation under the high salt concentration environment is not particularly limited, but for example, may be a period of approximately 1/3 of the entire cultivation period, a period of approximately 1/2 of the entire cultivation period, or a period of approximately 2/3 of the entire cultivation period, from immediately after the salt tolerance imparting treatment. From the viewpoint of improving the shelf life of the plant, it is preferable that the cultivation under the high salt concentration environment is performed throughout the entire cultivation period, from immediately after the salt tolerance imparting treatment.

If the seedling with only insufficient salt tolerance imparted is cultivated under the high salt concentration environment for a certain period in the cultivation step, the seedling withers. The withered plant causes the rotting, and allows undesirable bacteria or the like to proliferate in the nutrient solution. In such a case, despite the efforts made to impart the salt tolerance to the seedling, the seedling may wither by the disease or the like, due to contamination of the nutrient solution. Therefore, it is preferable that a removal step of removing the withered seedling is performed after the salt tolerance imparting step or during the cultivation step. Especially when the salt tolerance imparting step is performed by using the treatment solution having a sodium chloride concentration of 1 % by mass or more, it is preferable that the removal step is performed after the salt tolerance imparting step and before initiation of the cultivation step. In the cultivation of crops, the yield in an actual cultivation area can be improved by removing the withered seedling from the cultivation tank.

When plants are grown for a certain period under the high salt concentration environment after initiation of the salt tolerance imparting treatment, the seedling which has grown without withering can be confirmed to be a plant having the salt tolerance thereof surely improved by the salt tolerance imparting agent. By removing the withered seedling, the salt tolerant seedling produced according to the present invention can acquire quality assurance as a salt tolerant seedling.

The cultivation step may be performed indoors using the cultivation tank installed indoors, or may be performed outdoors in the open air using the cultivation tank installed outdoors.

When the plant is a fruit, the fruit is harvested from the plant cultivated until the harvest stage of the fruit. The plant production method of the present invention may further include a selection step of selecting, from the harvested fruits, those satisfying the above-mentioned respective ranges with respect to the sodium content, water content, or sugar content of the edible part of the fruit.

According to the plant production method of the present invention, the plant can be produced by using salt water or sea water.

### ≪Tolerance Imparting Method≫

The tolerance imparting method of the present invention include: a salt tolerance imparting step of performing a salt resistance imparting treatment by bringing a salt tolerance imparting agent into contact with at least a part of a root of a plant, thereby obtaining a salt tolerance imparted plant; a cultivation step of hydroponically cultivating the salt tolerance imparted plant obtained in the salt tolerance imparting step with a nutrient solution having a sodium chloride concentration of 1 % by mass or more. The salt tolerance imparting step is carried out for imparting salt tolerance to a plant originally having low salt tolerance by treating the plant with the salt tolerance imparting agent, thereby enabling the plant to be cultivated under an environment of very high salt concentration such as the sodium chloride concentration of 1 % by mass or more. In the subsequent cultivation step, the salt tolerance imparted plant can be grown by hydroponics with the nutrient solution having a sodium chloride concentration of 1 % by mass or more.

By performing the salt tolerance imparting step and the cultivation step, it is possible to impart tolerance to the plant against diseases and pests.

A plant at an early stage of growth has less stress tolerance than a sufficiently grown plant, and is more likely to be influenced by environmental stress. In particular, the process of rooting or budding is very sensitive to the salt concentration. Therefore, when grown under a high salt concentration environment from the stage of seeds or bulbs, many of the plants are caused to wither due to high salt stress before acquiring sufficient salt tolerance even if the salt tolerance imparting treatment is carried out. In the tolerance imparting method of the present invention, it is preferable that the plant is grown under a low salt concentration environment at an early stage of the growth, and the salt tolerance imparting treatment is performed after the plant has been grown to a certain extent. This can noticeably increase a proportion of the plants to which the salt tolerance is imparted by the salt tolerance imparting treatment, thereby making it possible to efficiently raise seedlings capable of being cultivated under a high salt concentration environment.

The tolerance imparting method of the present invention preferably includes, as an initial growth step, a step of allowing the plant to grow under an environment with a sodium chloride concentration of less than 1 % by mass, at least until the rooting and the budding are completed. The sodium chloride concentration of the environment in which the seed or the like is grown in the initial growth step may be less than 1 % by mass, and is preferably equal to or less than the salt concentration at which a plant of the same variety as the plant to be obtained by raising the seedling is capable of being normally grown. The environment that allows "capable of being normally grown" means an environment in which the cultivation of a plurality of plants results in a survival rate of 80 % or more. In the tolerance imparting method of the present invention, the sodium chloride concentration of the environment for the initial growth step is preferably 0 to 0.5 % by mass, more preferably 0 to 0.3 % by mass, and further preferably 0 to 0.1 % by mass.

The initial growth step can be performed by a general method for causing the seed to bud and root, except that an aqueous solution having a sodium chloride concentration of less than 1 % by mass is used as water (initial growth solution) to be supplied to the seed or the bulb. Specifically, the seed or the bulb is allowed to bud and root by placing the seed or the bulb in a state of being in contact with the initial growth solution under a temperature environment which allows budding and rooting. For example, the initial growth solution may be periodically sprayed onto the seed or the like which is placed under a suitable temperature environment. Alternatively, the seed or the like may be placed in a state where at least a part of the surface of the seed or the like is exposed to air while other parts are in contact with the initial growth solution under a suitable temperature environment. For example, the seed or the like may be placed on the surface of a support including the initial growth solution, thereby allowing the seed or the like to be in contact partially with the initial growth solution. Alternatively, the seed or the like is placed in the initial growth solution that is held in a container such that the surface of the solution is lower than the top of the seed or the like, to thereby allow the seed or the like to be in contact partially with the initial growth solution.

The support is not limited as long as the support has such a porosity that allows the initial growth solution contained therein to be supplied to the seed or the like placed on the surface of the carrier. However, it is preferable that the support has such a porosity that, after rooting, allows the root of the seedling to penetrate through the support. By allowing the plant budded and rooted from the seed or the like to grow such that a stem or a leaf grows upward from the support, and the root grows downward into the support, the plant can grow in a state of being supported by the support. For example, when the plant is grown such that the seed or the like is allowed to bud and root by placing the seed or the like on the surface of the support retained within a cultivation pot installable in a cultivation tank used for the hydroponics performed in the cultivation step, while allowing the root to grow downward into the support so as to penetrate through the support, it is possible, even after the seedling stage, to grow the plant by installing the cultivation pot as such in the cultivation tank, since the plant is supported in a state of being retained in the cultivation pot.

As a support having such porosity, for example, a gel material, a fiber-shaped material, or a granular or gravel-shaped material may be used. Examples of the gel material include polysaccharide polymers such as agar, agarose, gellan gum, and alginic acid; and water absorptive resins such as acrylic resin. Examples of the fiber-shaped material include non-woven fabric, cotton, paper, rock wool, and glass wool. Examples of the granular or gravel-shaped material include a wood chip, a bark, pumice, vermiculite, and sand.

After the initial growth step, the salt tolerance imparting treatment may be performed, as a salt tolerance imparting step, by bringing the salt tolerance imparting agent into contact with at least a part of a root of the grown seedling. The salt tolerance imparting treatment may be performed immediately after budding and rooting, but this treatment can enhance the tolerance against salt stress more as the seedling grows more. Therefore, it is preferable that the salt tolerance imparting step is performed after the seedling is grown for at least one week, and preferably for approximately three weeks, after budding.

The salt tolerance imparting treatment can be performed by putting at least a part of the root of the seedling into an aqueous solution (treatment solution) containing the salt tolerance imparting agent. The sodium chloride concentration of the treatment solution is not particularly limited, and may be appropriately adjusted depending on a kind of the salt tolerance imparting agent used or a kind of the plant such that a sufficient salt tolerance imparting efficiency is obtained. For example, the treatment solution may be a solution obtained by mixing the salt tolerance imparting agent with the initial growth solution, a solution obtained by mixing the salt tolerance imparting agent with the nutrient solution used in the cultivation step, or a solution having a salt composition different from those of the initial growth solution and the nutrient solution. The sodium chloride concentration of the treatment solution used in the present invention is preferably 1 % by mass or more, and more preferably the same as the sodium chloride concentration of the nutrient solution.

The salt tolerance imparting agent used in the present invention may be a drug, a microorganism, or a culture supernatant of the microorganism. Examples of the drug include pyrroloquinoline quinone (see Japanese Patent No. 5013326) and strigolactone. Examples of the microorganism include Paenibacillus Fukuinensis (see Japanese Unexamined Patent Application, First Publication No. 2013-75881). The salt tolerance imparting agent may be formed of one kind of microorganism, or a mixture of two or more kinds of microorganisms.

The concentration of the salt tolerance imparting agent in the treatment solution can be appropriately adjusted in consideration of the kind of the salt tolerance imparting agent, the kind of the plant, the growth stage or the like. When the concentration of the salt tolerance imparting agent in the treatment solution is too low, the salt tolerance imparting agent is less likely to get in contact with the root of the plant in the treatment solution, which may result in insufficient salt tolerance imparting effect. On the other hand, depending on the kind of the salt tolerance imparting agent, the growth of the plant may be adversely affected by excessive intake of the salt tolerance imparting agent. In view of this, an appropriate concentration of the salt tolerance imparting agent in the treatment solution for obtaining sufficient salt tolerance imparting effect can be determined empirically. For example, when the salt tolerance imparting agent is the microorganism, the concentration of the microorganism in the treatment solution may be set to be 10³ CFU/mL or more, whereby sufficient salt tolerance imparting effect can be obtained. When the salt tolerance imparting agent is the microorganism, the concentration of the microorganism in the treatment solution is preferably 10⁴ CFU/mL or more, and more preferably 10⁵ CFU/mL or more. When the salt tolerance imparting agent is the microorganism, an upper limit of the concentration of the microorganism in the treatment solution is not particularly limited, but for example, the concentration may be up to 10¹³ CFU/mL, whereby the quality of the treatment solution can be favorably maintained. The upper limit of the concentration of the microorganism in the treatment solution is preferably 10¹² CFU/mL, more preferably 10¹¹ CFU/mL, still more preferably 10¹⁰ CFU/mL, particularly preferably 10⁹ CFU/mL. The range of the concentration of the microorganism in the treatment solution may be, for example, 10³ to 10¹³ CFU/mL, preferably 10⁴ to 10¹² CFU/mL, more preferably 10⁵ to 10¹¹ CFU/mL, still more preferably 10⁵ to 10¹⁰ CFU/mL, especially preferably 10⁵ to 10⁹ CFU/mL.

The time period for performing the salt tolerance imparting treatment time once, that is, the time for holding at least a part of the root of the plant in the treatment solution, can be appropriately adjusted in consideration of the kind of the plant or the kind of the salt tolerance imparting agent to be used. For example, such time period for the salt tolerance imparting treatment is preferably 1 hour or more, more preferably 18 hours or more, still preferably one day or more, and further more preferably one day to seven days. Cultivating the plant for 1 hour or more with the root thereof being held in the treatment solution ensures sufficient chances for the salt tolerance imparting agent in the treatment solution to contact the root of the plant, whereby the salt tolerance can be imparted more easily.

In the initial growth step, when the seedling is to be grown in a state of being supported by the support retained within the cultivation pot, the cultivation pot may be installed in a treatment tank containing the treatment solution such that the root growing from below the support contacts the treatment solution, thereby carrying out the salt tolerance imparting treatment. For example, the root can be brought into contact with the treatment solution by using a float which has one or more through-holes for fitting the cultivation pot thereinto and is floated on the surface of the treatment solution, and fitting the cultivation pot into the float. The cultivation pot may be detachably fitted into the through-hole of the float, or may be fixed undetachably to the through-hole of the float. Alternatively, the float and the cultivation pot may be integrally formed. As a material of the float to be floated on the surface of the treatment solution, the same material as the float to be floated on the surface of the nutrient solution described later can be used.

The larger the amount of the treatment solution used in the salt tolerance imparting treatment, the larger the amount of the salt tolerance imparting agent needed. Therefore, by reducing the amount of the treatment solution to an amount that is necessary and sufficient to allow the root of the plant grown from the bottom surface of the cultivation pot to contact the treatment solution, the amount of the salt tolerance imparting agent required for performing the salt tolerance imparting treatment once can be suppressed. However, when the amount of the treatment solution is too small, it may become impossible to allow a sufficient amount of the salt tolerance imparting agent to contact the root of the plant. Therefore, when a plate into which one cultivation pot is fitted is installed per treatment tank, the amount of the treatment solution contained in the treatment tank is preferably at least 5 mL.

Thereafter, the seedling to which the salt tolerance is imparted by the salt tolerance imparting step is grown by hydroponics with the nutrient solution having a sodium chloride concentration of 1 % by mass or more.

In the present invention, the sodium chloride concentration of the nutrient solution in the cultivation step is not limited as long as the sodium chloride concentration is 1 % by mass or more, and may be appropriately adjusted in accordance with the salt tolerance of the plant to be cultivated. The sodium chloride concentration of the nutrient solution is preferably 1 to 4 % by mass, more preferably 1.5 to 3.8 % by mass, further preferably 2 to 3.5 % by mass, and particularly preferably 2.5 to 3.3 % by mass.

The nutrient solution used in the present invention preferably contains magnesium chloride in addition to sodium chloride, where the amount of magnesium chloride contained is preferably 0.5 % by mass or less, more preferably 0.1 to 0.5 % by mass.

In addition to sodium chloride and magnesium chloride, it is preferable that the nutrient solution used in the present invention contains various nutrient components which are necessary for the growth of the plant. The nutrient components can be appropriately adjusted according to the type of the plant to be cultivated. Especially, it is preferable that the nutrient solution contains elements necessary for the growth of the plant in the form of salts. Examples of such elements include nitrogen, phosphorus, potassium, calcium, magnesium, sulfur, iron, manganese, copper, molybdenum, and boron. The nutrient solution may further contain elements such as aluminum and silicon in the form of salts thereof, depending on the type of the plant. Further, the composition of the nutrient solution may be varied according to the growth stage of the plant.

The nutrient solution to be used in the present invention may be, for example, a solution prepared by supplementing deficient salt such as sodium chloride to commercially available liquid fertilizer or a solution obtained by diluting commercially available concentrated liquid fertilizer with sea water instead of water. Further, the nutrient solution may also be a solution obtained by appropriately adding a deficient salt such as salt of phosphorus to seawater.

In the present invention, the hydroponics in the cultivation step may be performed by a general hydroponics method, except that the sodium chloride concentration of the nutrient solution is set to 1 % by mass or more. The cultivation step may be performed by a deep flow technique in which a relatively large amount of the nutrient solution is stored in the cultivation tank, or a nutrient film technique in which a culture solution is allowed to flow down little by little on a flat surface having a gentle slope.

In the deep flow technique, the replacement of the nutrient solution in the cultivation tank may be carried out by a circulation method in which the nutrient solution used is circulated, or a non-circulation method in which the nutrient solution used for a certain period of time in the cultivation tank is discharged. In case of the circulation method, the nutrient solution prepared in a nutrient solution preparation tank is charged into the cultivation tank by a pump or the like, and is collected back to the nutrient solution preparation tank from the cultivation tank, and the nutrient component or the like is prepared.

For example, the deep flow technique can be carried out by using a hydroponic apparatus having a cultivation tank for storing the nutrient solution, a cultivation pot for accommodating the plant, one or more through-holes for fitting the cultivation pot, and a float to be floated on the surface of the nutrient solution. The cultivation pot may be detachably fitted into the through-hole of the float, or may be fixed undetachably to the through-hole of the float. Alternatively, the float and the cultivation pot may be integrally formed. The cultivation tank may be installed indoors, or may be installed outdoors.

In the case of a circulation type hydroponic cultivation apparatus, the cultivation tank includes a water supply hole for injecting the nutrient solution, and a drainage hole for draining the nutrient solution. In the case of a non-circulation type hydroponic apparatus, the cultivation tank may include both of the water supply hole and the drainage hole, or may include a water supply/drainage hole used for both of the water supply and the drainage. The water supply and drainage of the nutrient solution in the cultivation tank are controlled by a pump and a valve.

The cultivation pot is a container that has opening portions at least on an upper surface and a lower surface, and is capable of retaining the support. In general, the cultivation pot formed of a resin material such as polyethylene, polypropylene, or polyvinylidene chloride is used. As the support to be retained in the cultivation pot, any of those described above can be used.

The float is formed of a material that floats on the surface of the nutrient solution in a state where the cultivation pot being used for cultivating the plant is fitted into the through-hole. As such a material, for example, a foamed resin such as polystyrene foam or polypropylene foam is used. By fitting the cultivation pot into the float, the cultivation pot can be constantly positioned on the surface of the nutrient solution, regardless of a large or small amount of the nutrient solution, and even if the amount of the nutrient solution is small, the root of the plant can be allowed to contact constantly with the nutrient solution.

The float to be floated in the cultivation tank may be one sheet, or two or more sheets. When the cultivation tank is installed outdoors, it is preferable that the float is installed to cover most of the surface of the nutrient solution, in order to prevent evaporation of the nutrient solution from the surface thereof.

In the deep flow technique, it is preferable that the hydroponic apparatus used includes oxygen supply means for keeping the dissolved oxygen content of the nutrient solution at a predetermined level or higher. As the oxygen supply means, for example, an air pump or an air sucker can be used. By installing the air pump in the cultivation tank, air including oxygen can be directly supplied to the nutrient solution in the cultivation tank. When the air sucker is used, the nutrient solution can be charged into the cultivation tank after the nutrient solution is mixed with air by passing the nutrient solution through the air sucker or the like in advance.

The pH suitable for the hydroponics varies depending on the kind of the plant, and is generally approximately 5.5 to 6.5, but the pH of the nutrient solution tends to increase as the cultivation period extends longer. Therefore, in order to stably perform the hydroponics for a long period of time, it is preferable that the hydroponic apparatus used includes pH control means for measuring the pH of the nutrient solution periodically, and dosing an acid material in order to adjust the pH within a predetermined range as necessary. As the acid material used for the pH adjustment, for example, hydrochloric acid, sulfuric acid, or nitric acid can be used.

In the tolerance imparting method of the present invention, the salt tolerance imparting step and the cultivation step may be performed in the same cultivation tank, or the salt tolerance imparting step may performed in the treatment tank storing the treatment solution, followed by transferring the seedling after the treatment to the cultivation tank storing the nutrient solution.

In the initial growth step, when the salt tolerance imparting treatment is performed in the treatment tank after the seedling is grown in a state of being supported by the support retained within the cultivation pot, the cultivation pot with the seedling may be detached from the float of the treatment tank, and may be fitted into the through-hole of the float floating on the surface of the nutrient solution stored in the cultivation tank, or the float into which the cultivation pot is embedded may be transferred from the treatment tank to be floated on the surface of the nutrient solution in the cultivation tank. Transfer means for transferring the cultivation pot or the float to the cultivation tank from the treatment tank is not particularly limited, and for example, the transfer may be performed by means of a water current, or a conveyor. When a plurality of cultivation pots are installed per treatment tank, it is preferable that a bubbling treatment is performed by the air pump, in order to prevent stagnation of the treatment solution, and oxygen deficiency.

When the salt tolerance imparting treatment is performed in the cultivation tank, first, the treatment solution is charged to the cultivation tank, and the root grown downward in the cultivation pot fitted into the float is brought into contact with the treatment solution to thereby perform the salt tolerance imparting treatment. In order to prevent the occurrence of concentration gradient of the salt tolerance imparting agent, it is preferable that the treatment solution is contacted with the root of the plant while suppressing the amount of the water supply and drainage or without the water supply and drainage.

However, when the amount of the water supply and drainage is small or the water supply and drainage is not performed, the stagnation may occur in the cultivation tank, causing adverse effect on the plant itself. Therefore, it is preferable that the treatment solution is suitably stirred by the bubbling treatment with the air pump.

After the salt tolerance imparting treatment, the treatment solution in the cultivation tank is drained, and subsequently, the nutrient solution prepared in advance in another tank is supplied to the cultivation tank. Then, the cultivation step is initiated by supplying water and draining under normal conditions. When the salt tolerance imparting agent is composed of a material, such as the microorganism, which does not adversely affect the plant even in the event of excessive intake of the material, the nutrient solution may be supplied without draining the treatment solution, and the water supply and drainage may be initiated under the normal water supply and drainage conditions.

The cultivation with the nutrient solution having a sodium chloride concentration of 1 % by mass or more (hereinafter, also referred to as "under the high salt concentration environment") need not necessarily be performed throughout the entire cultivation period after the salt tolerance imparting treatment. For example, the cultivation under the high salt concentration environment may be performed only for an arbitrary period after the salt tolerance imparting treatment. In this case, it is preferable that the cultivation under the high salt concentration environment is performed for a predetermined period immediately after the salt tolerance imparting treatment. It is speculated that, by performing the cultivation under the high salt concentration environment at an arbitrary time immediately after the salt tolerance imparting treatment, the salt tolerance imparted by the salt tolerance imparting treatment is maintained, whereby the shelf life of the plant improves. The period of the cultivation under the high salt concentration environment is not particularly limited, but for example, may be a period of approximately 1/3 of the entire cultivation period, a period of approximately 1/2 of the entire cultivation period, or a period of approximately 2/3 of the entire cultivation period, from immediately after the salt tolerance imparting treatment. From the viewpoint of maintaining the tolerance against diseases and pests, it is preferable that the cultivation under the high salt concentration environment is performed throughout the entire cultivation period, from immediately after the salt tolerance imparting treatment.

If the seedling with only insufficient salt tolerance imparted is cultivated under the high salt concentration environment for a certain period in the cultivation step, the seedling withers. The withered plant causes the rotting, and allows undesirable bacteria or the like to proliferate in the nutrient solution. In such a case, despite the efforts made to impart the salt tolerance to the seedling, the seedling may wither by the disease or the like, due to contamination of the nutrient solution. Therefore, it is preferable that a removal step of removing the withered seedling is performed after the salt tolerance imparting step or during the cultivation step. Especially when the salt tolerance imparting step is performed by using the treatment solution having a sodium chloride concentration of 1 % by mass or more, it is preferable that the removal step is performed after the salt tolerance imparting step and before initiation of the cultivation step. In the cultivation of crops, the yield in an actual cultivation area can be improved by removing the withered seedling from the cultivation tank.

When plants are grown for a certain period under the high salt concentration environment after initiation of the salt tolerance imparting treatment, the seedling which has grown without withering can be confirmed to be a plant having the salt tolerance thereof surely improved by the salt tolerance imparting agent. By removing the withered seedling, the salt tolerant seedling produced according to the present invention can acquire quality assurance as a salt tolerant seedling.

The cultivation step may be performed indoors using the cultivation tank installed indoors, or may be performed outdoors in the open air using the cultivation tank installed outdoors. The plant cultivated in the cultivation step according to the present invention is also suitable for the cultivation performed outdoors in the open air, since the tolerance against diseases and pests is imparted to the plant.

According to the tolerance imparting method of the present invention, it is possible to impart tolerance to the plant against diseases and pests, and to achieve an effect of prevention, cure or extermination of pests and/or diseases. This is considered to be attributable to the fact that exposing the plant to the high salt concentration environment improves the tolerance against diseases and pests as well as the tolerance against salt stress.

In the present invention, the plant to which the tolerance against diseases and pests is imparted may be an angiosperm, a gymnosperm, or ferns or moss.

Further, the plant may be a monocotyledonous plant or a dicotyledonous plant. Specific examples of the plant include plants of the Gramineae family such as rice, corn, sorghum, wheat, barley, rye, barnyard millet, or foxtail millet; the plant of the Solanaceae family such as tomato, eggplant, paprika, bell pepper, potato, or tobacco; plants of the Brassicaceae family such as Arabidopsis thaliana, colza, shepherd's purse, radish, cabbage, violet cabbage, Brussels sprouts (Petit vert), Chinese cabbage, bok choy, kale, watercress, Japanese mustard spinach, broccoli, cauliflower, turnip, Japanese horseradish, or mustard; plants of the Cucurbitaceae family such as cucumber, bitter melon, pumpkin, melon, or watermelon; plants of the Vitaceae family such as grape; plants of the Rutaceae family such as lemon, orange, navel orange, grapefruit, mandarin orange, lime, Citrus sudachi, citron, Shikuwasa, or citrus tankan; plants of the Rosaceae family such as apple, cherry, Japanese apricot, peach, loquat, apricot, plum (Prumus salicina), prune, almond, pear, European pear, strawberry, raspberry, blackberry, black currant, cranberry, or blueberry; plants of the Leguminosae family such as soybean, kidney bean, pea, broad bean, green soybean, green gram, or chick pea; plants of the Nelumbonaceae family such as lotus (lotus root); plants of the Pedaliaceae family such as sesame; plants of the Chenopodiaceae family such as spinach, beet, sugar beet, quinoa, tumbleweed, amaranth, or cockscomb; plants of the Arecaceae family such as date palm, oil palm, coconut, or acai; plants of the Musaceae family such as banana, Musa basjoo, or Manila hemp; plants of the Malvaceae family such as cotton or okra; plants of the Myrtaceae family such as eucalyptus; and plants of the Capparaceae family such as Cleome gynandra or Cleome spinose.

Among these plants, the plants of the Solanaceae family are preferable, and tomato (Solanum lycopersicum) is more preferable.

In the present invention, the pests against which the tolerance of the tolerance imparted plant is exerted include all organisms which harm the growth of the plant, and the examples thereof include aphids such as a cotton aphid (Aphis gossypii), a green peach aphid (Myzus persicae), and Kaltenbach (Aulacorthum solani), leafminer flies such as an eggplant leafminer fly (Liriomyza bryoniae), a bean leafminer fly (Liriomyza trifolii), and a tomato leafminer fly (Liriomyza sativae), whiteflies such as a greenhouse whitefly (Trialeurodes vaporariorum), a tobacco whitefly (Bemisia tabaci), and a silver leaf whitefly (Bemisia argentifolii), thrips such as a western flow thrip (Thrips parmi), a Japanese flow thrip (Thrips setosus), and an Intonsa flower thrip (Frankliniella intonsa), and rust mites such as a tomato rust mite (Aculops lycopersici).

In the present invention, the pathogens against which the tolerance of the tolerance imparted plant is exerted include all organisms which may cause diseases of the plant, and the examples thereof include fungi, oomycetes, bacteria, virus, and the like.

Regarding these pathogens, it is preferable that the plant is imparted with tolerance against a viral disease in which the pathogen is a virus or a fungal disease in which the pathogen is fungi. There are many viral diseases and fungal diseases for which effective agricultural chemicals are scarce and the prevention or extermination of pathogen is difficult. However, according to the tolerance imparting method of the present invention, it is possible to easily impart the tolerance to the plant against viral diseases or fungal diseases.

Examples of viral disease of tomato include a tomato yellow leaf curl disease (Tomato yellow leaf curl virus: TYLCV), a tomato mosaic disease (Tomato mosaic virus: ToMV), and a tomato spotted wilt disease (Tomato spotted wilt virus: TSWV). Examples of fungal disease of tomato include tomato powdery mildew.

According to the tolerance imparting method of the present invention, it is possible to impart the tolerance to the plant by using salt water or sea water. Therefore, the amount of the agricultural chemical used hitherto for preventing diseases and pests can be reduced, and it becomes possible to realize a cultivation method that is friendly to natural environment.

### ≪Tomato≫

The tomato of the present invention satisfies at least one of the following (1) to (20), which respectively represent amounts of free amino acids contained per 100 g of edible portion of a fruit of the tomato:
(1) free glutamic acid : 200 mg or more,
(2) free aspartic acid : 40 mg or more,
(3) free arginine : 6 mg or more,
(4) free isoleucine : 6 mg or more,
(5) free alanine : 8 mg or more,
(6) free serine : 15 mg or more,
(7) free lysine : 7 mg or more,
(8) free histidine : 7 mg or more,
(9) free phenylalanine : 12 mg or more,
(10) free tyrosine : 4 mg or more,
(11) free leucine : 4 mg or more,
(12) free methionine : 2 mg or more,
(13) free valine : 3.5 mg or more,
(14) free glycine : 2 mg or more,
(15) free proline : 50 mg or less,
(16) free threonine : 10 mg or more,
(17) free tryptophan : 2 mg or more,
(18) free phosphoserine : 1.2 mg or more,
(19) free β-alanine : 2 mg or more, and
(20) free γ-aminobutyric acid : 80 mg or more.

With respect to the amounts of free amino acids contained per 100 g of edible portion of a fruit of the tomato, it is preferable that the tomato satisfies at least one of (1), (2), (4) to (6), (8), (13), (14) and (16), and it is more preferable that the tomato satisfies at least one of (1), (5), (6), (14) and (16). With respect to the amounts of free amino acids contained per 100 g of edible portion of a fruit of the tomato, it is still more preferable that the tomato satisfies all of (1), (5), (6), (14) and (16), and it is particularly preferable that the tomato satisfies all of (1), (2), (4) to (6), (8), (13), (14) and (16) .

With respect to the amount of free glutamic acid contained per 100 g of edible portion of a fruit of the tomato, the amount is preferably 200 mg or more, more preferably 300 mg or more, and still more preferably 500 mg or more. The upper limit of the amount of free glutamic acid contained per 100 g of edible portion of a fruit of the tomato is not particularly limited, and for example, may be 2000 mg, 800 mg, 700 mg or 600 mg. With respect to the range of the amount of free glutamic acid contained per 100 g of edible portion of a fruit of the tomato, for example, the amount is preferably 200 to 2000 mg, more preferably 300 to 800 mg, still more preferably 400 to 700 mg, and particularly preferably 500 to 600 mg.

Glutamic acid is a flavor ingredient and gives a good taste to the fruit of tomato. In addition, glutamic acid is an excitatory neurotransmitter and effective for fatigue recovery.

With respect to the amount of free aspartic acid contained per 100 g of edible portion of a fruit of the tomato, the amount is preferably 50 mg or more, more preferably 70 mg or more, still more preferably 85 mg or more, and particularly preferably 100 mg or more. The upper limit of the amount of free aspartic acid contained per 100 g of edible portion of a fruit of the tomato is not particularly limited, and for example, may be 300 mg, 200 mg, 180 mg or 150 mg. With respect to the range of the amount of free aspartic acid contained per 100 g of edible portion of a fruit of the tomato, for example, the amount is preferably, 40 to 300 mg, more preferably 50 to 300 mg, still more preferably 70 to 200 mg, further more preferably 85 to 180 mg, and particularly preferably 100 to 150 mg.

Aspartic acid is effective for fatigue recovery, and is also a fast-acting energy source.

With respect to the amount of free arginine acid contained per 100 g of edible portion of a fruit of the tomato, the amount is preferably 6 mg or more, more preferably 10 mg or more, still more preferably 15 mg or more, and particularly preferably 20 mg or more. The upper limit of the amount of free arginine acid contained per 100 g of edible portion of a fruit of the tomato is not particularly limited, and for example, may be 100 mg, 50 mg, or 30 mg. With respect to the range of the amount of free arginine acid contained per 100 g of edible portion of a fruit of the tomato, for example, the amount is preferably 6 to 150 mg, preferably 10 to 150 mg, more preferably 10 to 100 mg, still more preferably 15 to 100 mg, and particularly preferably 20 to 50 mg.

Arginine has an effect of improving immune function, vasodilating action and the like.

With respect to the amount of free isoleucine contained per 100 g of edible portion of a fruit of the tomato, the amount is preferably 6 mg or more, more preferably 7 mg or more, still more preferably 8 mg or more, further preferably 10 mg or more, and particularly preferably 15 mg or more. The upper limit of the amount of free isoleucine contained per 100 g of edible portion of a fruit of the tomato is not particularly limited, and for example, may be 100 mg, 80 mg, 50 mg or 30 mg. With respect to the range of the amount of free isoleucine contained per 100 g of edible portion of a fruit of the tomato, for example, the amount is preferably 6 to 100 mg, more preferably 7 to 100 mg, still more preferably 10 to 80 mg, further preferably 13 to 50 mg, and particularly preferably 15 to 30 mg.

Isoleucine is an essential amino acid and serves as an energy source. Also, isoleucine has effects such as vasodilation, growth promotion, muscle strengthening, fatigue recovery and the like.

With respect to the amount of free alanine contained per 100 g of edible portion of a fruit of the tomato, the amount is preferably 8 mg or more, more preferably 9 mg or more, still more preferably 10 mg or more, still more preferably 20 mg or more, further more preferably 40 mg or more, and particularly preferably 50 mg or more. The upper limit of the amount of free alanine contained per 100 g of edible portion of a fruit of the tomato is not particularly limited, and for example, may be 200 mg, 150 mg, 100 mg or 80 mg. With respect to the range of the amount of free alanine contained per 100 g of edible portion of a fruit of the tomato, for example, the amount is preferably 8 to 200 mg, more preferably 10 to 150 mg, still more preferably 20 to 150 mg, further more preferably 40 to 100 mg, and particularly preferably 50 to 80 mg.

Alanine serves as an energy source and has effects such as improvement of immune function and improvement of activity of liver (alcohol decomposition) and the like.

With respect to the amount of free serine contained per 100 g of edible portion of a fruit of the tomato, the amount is preferably 15 mg or more, more preferably 30 mg or more, still more preferably 40 mg or more, still more preferably 50 mg or more, further more preferably 70 mg or more, and particularly preferably 90 mg or more. The upper limit of the amount of free serine contained per 100 g of edible portion of a fruit of the tomato is not particularly limited, and for example, may be 300 mg, 250 mg, 200 mg or 150 mg. With respect to the range of the amount of free serine contained per 100 g of edible portion of a fruit of the tomato, for example, the amount is preferably 15 to 300 mg, more preferably 30 to 250 mg, still more preferably 40 to 250 mg, still more preferably 50 to 200 mg, further more preferably 70 to 200 mg, and particularly preferably 90 to 150 mg.

Serine has effects of improving sleep quality, brain function assistance, and the like.

With respect to the amount of free lysine contained per 100 g of edible portion of a fruit of the tomato, the amount is preferably 7 mg or more, more preferably 10 mg or more, still more preferably 15 mg or more, and particularly preferably 20 mg or more. The upper limit of the amount of free lysine contained per 100 g of edible portion of a fruit of the tomato is not particularly limited, and for example, may be 100 mg, 50 mg, or 30 mg. With respect to the range of the amount of free lysine contained per 100 g of edible portion of a fruit of the tomato, for example, the amount is preferably 7 to 100 mg, more preferably 10 to 100 mg, still more preferably 15 to 50 mg, and particularly preferably 20 to 30 mg.

Lysine is an essential amino acid, and has body tissue repairing action. The deficiency of this amino acid causes growth disturbance. Lysine also has a virus suppressing effect.

With respect to the amount of free histidine contained per 100 g of edible portion of a fruit of the tomato, the amount is preferably 7 mg or more, more preferably 10 mg or more, still more preferably 15 mg or more, and particularly preferably 20 mg or more. The upper limit of the amount of free histidine contained per 100 g of edible portion of a fruit of the tomato is not particularly limited, and for example, may be 100 mg, 80 mg, or 60 mg. With respect to the range of the amount of free histidine contained per 100 g of edible portion of a fruit of the tomato, for example, the amount is preferably 7 to 100 mg, more preferably 10 to 100 mg, still more preferably 15 to 80 mg, and particularly preferably 20 to 60 mg.

Histidine is an essential amino acid and has leukopoietic/erythropoietic action. Histidine also has an anorectic effect.

With respect to the amount of free phenylalanine contained per 100 g of edible portion of a fruit of the tomato, the amount is preferably 12 mg or more, more preferably 20 mg or more, still more preferably 23 mg or more, and particularly preferably 25 mg or more. The upper limit of the amount of free phenylalanine contained per 100 g of edible portion of a fruit of the tomato is not particularly limited, and for example, may be 100 mg, 80 mg, or 60 mg. With respect to the range of the amount of free phenylalanine contained per 100 g of edible portion of a fruit of the tomato, for example, the amount is preferably 12 to 100 mg, more preferably 20 to 100 mg, still more preferably 23 to 80 mg, and particularly preferably 25 to 60 mg.

Phenylalanine is an essential amino acid and has analgesic action, antidepressant action, memory improvement effect and the like.

With respect to the amount of free tyrosine contained per 100 g of edible portion of a fruit of the tomato, the amount is preferably 4 mg or more, more preferably 7 mg or more, and still more preferably 9 mg or more. The upper limit of the amount of free tyrosine contained per 100 g of edible portion of a fruit of the tomato is not particularly limited, and for example, may be 100 mg or 50 mg. With respect to the range of the amount of free tyrosine contained per 100 g of edible portion of a fruit of the tomato, for example, the amount is preferably 4 to 100 mg, more preferably 7 to 100 mg, and still more preferably 9 to 50 mg.

Tyrosine is a precursor of a neurotransmitter and the like, and has an effect such as concentration improvement.

With respect to the amount of free leucine contained per 100 g of edible portion of a fruit of the tomato, the amount is preferably 4 mg or more, more preferably 6 mg or more, and still more preferably 7 mg or more. The upper limit of the amount of free leucine contained per 100 g of edible portion of a fruit of the tomato is not particularly limited, and for example, may be 100 mg or 50 mg. With respect to the range of the amount of free leucine contained per 100 g of edible portion of a fruit of the tomato, for example, the amount is preferably 4 to 100 mg, more preferably 7 to 100 mg, and still more preferably 7 to 50 mg.

Leucine is an essential amino acid and serves as an energy source. Leucine also has effects such as muscle strengthening and liver function improvement.

With respect to the amount of free methionine contained per 100 g of edible portion of a fruit of the tomato, the amount is preferably 2 mg or more. The upper limit of the amount of free methionine contained per 100 g of edible portion of a fruit of the tomato is not particularly limited, and for example, may be 100 mg or 50 mg.

With respect to the range of the amount of free methionine contained per 100 g of edible portion of a fruit of the tomato, for example, the amount is preferably 2 to 100 mg, and more preferably 2 to 50 mg.

Methionine is an essential amino acid and has allergic reaction suppressing effect, depressive effect, liver/kidney function improving effect, and the like.

With respect to the amount of free valine contained per 100 g of edible portion of a fruit of the tomato, the amount is preferably 3.5 mg or more, and more preferably 7 mg or more. The upper limit of the amount of free valine contained per 100 g of edible portion of a fruit of the tomato is not particularly limited, and for example, may be 100 mg or 50 mg. With respect to the range of the amount of free valine contained per 100 g of edible portion of a fruit of the tomato, for example, the amount is preferably 3.5 to 100 mg, more preferably 7 to 100 mg, and still more preferably 7 to 50 mg.

Valine is an essential amino acid and serves as an energy source. Valine also has effects such as growth promotion and liver function improvement.

With respect to the amount of free glycine contained per 100 g of edible portion of a fruit of the tomato, the amount is preferably 2 mg or more, more preferably 3 mg or more, still more preferably 5 mg or more, and particularly preferably 7 mg or more. The upper limit of the amount of free glycine contained per 100 g of edible portion of a fruit of the tomato is not particularly limited, and for example, may be 100 mg, 50 mg, or 30 mg. With respect to the range of the amount of free glycine contained per 100 g of edible portion of a fruit of the tomato, for example, the amount is preferably 2 to 100 mg, more preferably 5 to 50 mg, and still more preferably 7 to 30 mg.

Glycine has effects of improving sleep quality and the like.

With respect to the range of the amount of free proline contained per 100 g of edible portion of a fruit of the tomato, for example, the amount is preferably 50 mg or less, more preferably 1 to 50 mg, still more preferably 5 to 40 mg, and particularly preferably 10 to 30 mg.

Proline has effects of ameliorating joint pain, beautifying skin and the like, and is also a natural moisturizing ingredient.

With respect to the amount of free threonine contained per 100 g of edible portion of a fruit of the tomato, the amount is preferably 10 mg or more, more preferably 15 mg or more, and still more preferably 23 mg or more. The upper limit of the amount of free threonine contained per 100 g of edible portion of a fruit of the tomato is not particularly limited, and for example, may be 100 mg, 80 mg, or 50 mg. With respect to the range of the amount of free threonine contained per 100 g of edible portion of a fruit of the tomato, for example, the amount is preferably 10 to 100 mg, more preferably 15 to 80 mg, and still more preferably 23 to 50 mg.

Threonine is an essential amino acid and has effects such as growth promotion and liver function improvement.

With respect to the range of the amount of free tryptophan contained per 100 g of edible portion of a fruit of the tomato, for example, the amount is preferably 2 mg or more, and more preferably 2 to 50 mg.

Tryptophan is an essential amino acid and has a tranquilizing action as a serotonin precursor. Tryptophan also has a sedative effect, a sleep improving effect, and the like.

With respect to the amount of free phosphoserine contained per 100 g of edible portion of a fruit of the tomato, the amount is preferably 1.2 mg or more, and more preferably 1.5 mg or more. The upper limit of the amount of free phosphoserine contained per 100 g of edible portion of a fruit of the tomato is not particularly limited, and for example, may be 20 mg, 10 mg, or 5 mg. With respect to the range of the amount of free phosphoserine contained per 100 g of edible portion of a fruit of the tomato, for example, the amount is preferably 1.2 to 20 mg, more preferably 1.2 to 10 mg, and still more preferably 1.5 to 5 mg.

Phosphoserine is a precursor of serine, and has effects of improving sleep quality, brain function assistance and the like as in the case of serine.

With respect to the amount of free β-alanine contained per 100 g of edible portion of a fruit of the tomato, the amount is preferably 2 mg or more, more preferably 3 mg or more, and still more preferably 4 mg or more. The upper limit of the amount of free β-alanine contained per 100 g of edible portion of a fruit of the tomato is not particularly limited, and for example, may be 20 mg, 10 mg, or 8 mg. With respect to the range of the amount of free β-alanine contained per 100 g of edible portion of a fruit of the tomato, for example, the amount is preferably 2 to 20 mg, more preferably 2 to 10 mg, still more preferably 3 to 10 mg, and particularly preferably 4 to 8 mg.

β-alanine has effects such as suppression of fatigue and cognitive function improvement.

With respect to the amount of free γ-aminobutyric acid contained per 100 g of edible portion of a fruit of the tomato, the amount is preferably 80 mg or more, more preferably 100 mg or more, and still more preferably 150 mg or more. The upper limit of the amount of free γ-aminobutyric acid contained per 100 g of edible portion of a fruit of the tomato is not particularly limited, and for example, may be 500 mg, 300 mg, or 250 mg. With respect to the range of the amount of free γ-aminobutyric acid contained per 100 g of edible portion of a fruit of the tomato, for example, the amount is preferably 80 to 500 mg, more preferably 100 to 300 mg, still more preferably 100 to 250 mg, and particularly preferably 150 to 250 mg.

γ-aminobutyric acid has brain function improving effect, blood pressure improving effect, and the like.

With respect to the total amount of free proteogenic amino acids excluding asparagine and glutamine contained per 100 g of edible portion of a fruit of the tomato, the total amount is preferably 300 mg or more, more preferably 400 mg or more, and still more preferably 500 mg or more. The upper limit of the total amount of free proteogenic amino acids excluding asparagine and glutamine contained per 100 g of edible portion of a fruit of the tomato is not particularly limited, and for example, may be 3000 mg or 2000 mg. With respect to the range of the total amount of free proteogenic amino acids excluding asparagine and glutamine contained per 100 g of edible portion of a fruit of the tomato, for example, the amount is preferably 300 to 3000 mg, more preferably 400 to 3000 mg, and still more preferably 400 to 200 mg.

For example, in the case of a tomato grown by hydroponics by the tomato production method described below with a cultivating solution having a sodium chloride concentration of 1 mass% or more, the tomato preferably satisfies at least one of the following (1) to (12), which respectively represent amounts of free amino acids contained per 100 g of edible portion of a fruit of the tomato:
(1) free glutamic acid : 200 mg or more, preferably 300 mg or more,
(2) free aspartic acid : 40 mg or more,
(3) free isoleucine : 6 mg or more,
(4) free alanine : 8 mg or more,
(5) free serine : 15 mg or more, preferably 30 mg or more,
(6) free histidine : 7 mg or more,
(7) free valine : 3.5 mg or more, preferably 4 mg or more,
(8) free glycine : 2 mg or more,
(9) free threonine : 10 mg or more,
(10) free phosphoserine : 1.2 mg or more,
(11) free β-alanine : 2 mg or more, preferably 3 mg or more, and
(12) free γ-aminobutyric acid : 80 mg or more, preferably 100 mg or more, more preferably 150 mg or more.

With respect to the above (1) to (12), it is preferable that 5 or more of (1) to (12) are satisfied, it is more preferable that 10 or more of (1) to (12) are satisfied, and it is still more preferable that all of (1) to (12) are satisfied.

With respect to the total amount of free proteogenic amino acids excluding asparagine and glutamine contained per 100 g of edible portion of a fruit of the tomato, the total amount is preferably 300 mg or more, and more preferably 400 mg or more.

For example, in the case of a tomato grown by hydroponics by the tomato production method described below with a cultivating solution having a sodium chloride concentration of 2 mass% or more, the tomato preferably satisfies at least one of the following (1) to (15), which respectively represent amounts of free amino acids contained per 100 g of edible portion of a fruit of the tomato:
(1) free glutamic acid : 200 mg or more, preferably 300 mg or more, more preferably 500 mg or more,
(2) free aspartic acid : 40 mg or more, preferably 50 mg or more, more preferably 70 mg or more,
(3) free arginine : 6 mg or more,
(4) free isoleucine : 6 mg or more, preferably 7 mg or more, more preferably 8 mg or more,
(5) free alanine : 8 mg or more, preferably 9 mg or more, more preferably 10 mg or more, still more preferably 20 mg or more,
(6) free serine : 15 mg or more, preferably 30 mg or more, more preferably 40 mg or more, still more preferably 50 mg or more,
(7) free lysine : 7 mg or more,
(8) free histidine : 7 mg or more, preferably 10 mg or more,
(9) free leucine : 4 mg or more,
(10) free valine : 3.5 mg or more,
(11) free glycine : 2 mg or more,
(12) free threonine : 10 mg or more, preferably 15 mg or more,
(13) free phosphoserine : 1.2 mg or more, preferably 1.5 mg or more,
(14) free β-alanine : 2 mg or more, preferably 3 mg or more, more preferably 4 mg or more.
(15) free γ-aminobutyric acid : 80 mg or more, preferably 100 mg or more, more preferably 150 mg or more.

With respect to the above (1) to (15), it is preferable that 5 or more of (1) to (15) are satisfied, it is more preferable that 10 or more of (1) to (15) are satisfied, and it is still more preferable that all of (1) to (15) are satisfied.

With respect to the total amount of free proteogenic amino acids excluding asparagine and glutamine contained per 100 g of edible portion of a fruit of the tomato, the total amount is preferably 500 mg or more, more preferably 600 mg or more, still more preferably 800 mg or more, and particularly preferably 900 mg or more.

For example, in the case of a tomato grown by hydroponics by the tomato production method described below with a cultivating solution having a sodium chloride concentration of 3 mass% or more, the tomato preferably satisfies at least one of the following (1) to (16), which respectively represent amounts of free amino acids contained per 100 g of edible portion of a fruit of the tomato:
(1) free glutamic acid : 200 mg or more, preferably 300 mg or more, more preferably 500 mg or more,
(2) free aspartic acid : 40 mg or more, preferably 50 mg or more, more preferably 70 mg or more, still more preferably 85 mg or more, particularly preferably 100 mg or more,
(3) free arginine : 6 mg or more, preferably 10 mg or more,
(4) free isoleucine : 6 mg or more, preferably 7 mg or more, more preferably 8 mg or more, still more preferably 10 mg or more,
(5) free alanine : 8 mg or more, preferably 9 mg or more, more preferably 10 mg or more,
(6) free serine : 15 mg or more, preferably 30 mg or more, more preferably 40 mg or more, still more preferably 50 mg or more, particularly preferably 70 mg or more,
(7) free lysine : 7 mg or more, preferably 10 mg or more,
(8) free histidine : 7 mg or more, preferably 10 mg or more, more preferably 15 mg or more, still more preferably 20 mg or more,
(9) free phenylalanine : 12 mg or more,
(10) free tyrosine : 4 mg or more,
(11) free leucine : 4 mg or more, preferably 6 mg or more,
(12) free methionine : 2 mg or more,
(13) free valine : 3.5 mg or more, preferably 7 mg or more,
(14) free glycine : 2 mg or more, preferably 3 mg or more,
(15) free threonine : 10 mg or more, preferably 15 mg or more, more preferably 23 mg or more, and
(16) free tryptophan : 2 mg or more.

With respect to the above (1) to (16), it is preferable that 5 or more of (1) to (16) are satisfied, it is more preferable that 10 or more of (1) to (16) are satisfied, and it is still more preferable that all of (1) to (16) are satisfied.

With respect to the total amount of free proteogenic amino acids excluding asparagine and glutamine contained per 100 g of edible portion of a fruit of the tomato, the total amount is preferably 500 mg or more, more preferably 600 mg or more, still more preferably 800 mg or more, and particularly preferably 900 mg or more.

The mass ratio of free glutamic acid/free proline, which are contained per 100 g of edible portion of a fruit of the tomato, is preferably 1 or more, more preferably 1 to 30, still more preferably 5 to 27, and further more preferably 10 to 25.

The mass ratio of free aspartic acid/free proline, which are contained per 100 g of edible portion of a fruit of the tomato, is preferably 0.5 or more, more preferably 0.5 to 10, still more preferably 0.7 to 7, and further more preferably 1 to 5.

The amounts of amino acids can be measured by a known method. For example, a commercially available amino acid automatic analyzer can be used, and the amounts of amino acids can be determined by an amino acid automatic analysis method or a high performance liquid chromatography.

The tomato of the present invention has unique flavor due to the aforementioned specific contents or ratios of amino acids.

The tomato of the present invention preferably has a sodium content of 0.15 % by mass or more as measured with respect to edible portion of a fruit of the tomato. The sodium content is more preferably 0.18 to 0.4 % by mass, and still more preferably 0.2 to 0.3 % by mass. The sodium content can be measured by known method.

The tomato of the present invention preferably has a water content of 90 % by mass or less as measured with respect to edible portion of a fruit of the plant. The water content is more preferably 80 to 90 % by mass, still more preferably 83 to 89 % by mass, and still more preferably 85 to 88% by mass. The water content can be measured by known method. For example, the water content can be determined by a heat drying method using a commercially available dryer.

The tomato of the present invention preferably has a sugar content (Brix) of 8 or more as measured with respect to edible portion of a fruit of the tomato. The sugar content (Brix) is more preferably 8 to 20, still more preferably 10 to 15, and still more preferably 12 to 14. The sugar content can be measured by known method. For example, the sugar content can be measured using a commercially available sugar content refractometer.

The edible part of the fruit means a part of the harvested fruit excluding calyx and stem. The fruit is an unprocessed raw fruit.

With respect to the fruit of the tomato of the present invention, the amounts of free amino acids, the sodium content, the water content, and the sugar content can be determined by measuring the respective amounts with respect to the whole edible part of the fruit.

As regards the species of the tomato of the present invention, tomato (Solanum lycopersicum) is preferable.

The degree of ripening of tomato can be classified by the extent (area%) of red or pink coloration on the fruit surface. For example, a tomato goes through a green ripe period (no coloration), a degreening period (up to 70 % coloration), a mature period (71 to 90 % coloration), and a ripe period (91 to 100 % coloration) to reach an over-ripe period. With respect to the fruit of the tomato of the present invention, it is preferable that the amounts of free amino acids, the sodium content, the water content and the sugar content are within the aforementioned ranges in the mature period or the ripe period.

### ≪ Tomato Production Method ≫

The tomato production method of the present invention includes:
a salt tolerance imparting step of performing a salt resistance imparting treatment by bringing a salt tolerance imparting agent into contact with at least a part of a root of a tomato, thereby obtaining a salt tolerance imparted tomato; and
a cultivation step of hydroponically cultivating the salt tolerance imparted tomato obtained in the salt tolerance imparting step with a nutrient solution having a sodium chloride concentration of 1 % by mass or more. The salt tolerance imparting step is carried out for imparting salt tolerance to a plant originally having low salt tolerance by treating the tomato with the salt tolerance imparting agent, thereby enabling the tomato to be cultivated under an environment of very high salt concentration such as the sodium chloride concentration of 1 % by mass or more. In the subsequent cultivation step, the salt tolerance imparted tomato can be grown by hydroponics with the nutrient solution having a sodium chloride concentration of 1 % by mass or more.

By performing the salt tolerance imparting step and the cultivation step, the tomato of the present invention can be produced.

A plant at an early stage of growth has less stress tolerance than a sufficiently grown plant, and is more likely to be influenced by environmental stress. In particular, the process of rooting or budding is very sensitive to the salt concentration. Therefore, when grown under a high salt concentration environment from the stage of seeds or bulbs, many of the plants are caused to wither due to high salt stress before acquiring sufficient salt tolerance even if the salt tolerance imparting treatment is carried out. In the tomato production method of the present invention, it is preferable that the tomato is grown under a low salt concentration environment at an early stage of the growth, and the salt tolerance imparting treatment is performed after the tomato has been grown to a certain extent. This can noticeably increase a proportion of the plants to which the salt tolerance is imparted by the salt tolerance imparting treatment, thereby making it possible to efficiently raise seedlings capable of being cultivated under a high salt concentration environment.

The tomato production method of the present invention preferably includes, as an initial growth step, a step of allowing the tomato to grow under an environment with a sodium chloride concentration of less than 1 % by mass, at least until the rooting and the budding are completed. The sodium chloride concentration of the environment in which the seed or the like is grown in the initial growth step may be less than 1 % by mass, and is preferably equal to or less than the salt concentration at which a plant of the same variety as the plant to be obtained by raising the seedling is capable of being normally grown. The environment that allows "capable of being normally grown" means an environment in which the cultivation of a plurality of plants results in a survival rate of 80 % or more. In the tomato production method of the present invention, the sodium chloride concentration of the environment for the initial growth step is preferably 0 to 0.5 % by mass, more preferably 0 to 0.3 % by mass, and further preferably 0 to 0.1 % by mass.

The initial growth step can be performed by a general method for causing the seed to bud and root, except that an aqueous solution having a sodium chloride concentration of less than 1 % by mass is used as water (initial growth solution) to be supplied to the seed or the bulb. Specifically, the seed or the bulb is allowed to bud and root by placing the seed or the bulb in a state of being in contact with the initial growth solution under a temperature environment which allows budding and rooting. For example, the initial growth solution may be periodically sprayed onto the seed or the like which is placed under a suitable temperature environment. Alternatively, the seed or the like may be placed in a state where at least a part of the surface of the seed or the like is exposed to air while other parts are in contact with the initial growth solution under a suitable temperature environment. For example, the seed or the like may be placed on the surface of a support including the initial growth solution, thereby allowing the seed or the like to be in contact partially with the initial growth solution. Alternatively, the seed or the like is placed in the initial growth solution that is held in a container such that the surface of the solution is lower than the top of the seed or the like, to thereby allow the seed or the like to be in contact partially with the initial growth solution.

The support is not limited as long as the support has such a porosity that allows the initial growth solution contained therein to be supplied to the seed or the like placed on the surface of the carrier. However, it is preferable that the support has such a porosity that, after rooting, allows the root of the seedling to penetrate through the support. By allowing the plant budded and rooted from the seed or the like to grow such that a stem or a leaf grows upward from the support, and the root grows downward into the support, the plant can grow in a state of being supported by the support. For example, when the plant is grown such that the seed or the like is allowed to bud and root by placing the seed or the like on the surface of the support retained within a cultivation pot installable in a cultivation tank used for the hydroponics performed in the cultivation step, while allowing the root to grow downward into the support so as to penetrate through the support, it is possible, even after the seedling stage, to grow the plant by installing the cultivation pot as such in the cultivation tank, since the plant is supported in a state of being retained in the cultivation pot.

As a support having such porosity, for example, a gel material, a fiber-shaped material, or a granular or gravel-shaped material may be used. Examples of the gel material include polysaccharide polymers such as agar, agarose, gellan gum, and alginic acid; and water absorptive resins such as acrylic resin. Examples of the fiber-shaped material include non-woven fabric, cotton, paper, rock wool, and glass wool. Examples of the granular or gravel-shaped material include a wood chip, a bark, pumice, vermiculite, and sand.

After the initial growth step, the salt tolerance imparting treatment may be performed, as a salt tolerance imparting step, by bringing the salt tolerance imparting agent into contact with at least a part of a root of the grown seedling. The salt tolerance imparting treatment may be performed immediately after budding and rooting, but this treatment can enhance the tolerance against salt stress more as the seedling grows more. Therefore, it is preferable that the salt tolerance imparting step is performed after the seedling is grown for at least one week, and preferably for approximately three weeks, after budding.

The salt tolerance imparting treatment can be performed by putting at least a part of the root of the seedling into an aqueous solution (treatment solution) containing the salt tolerance imparting agent. The sodium chloride concentration of the treatment solution is not particularly limited, and may be appropriately adjusted depending on a kind of the salt tolerance imparting agent used or a kind of the plant such that a sufficient salt tolerance imparting efficiency is obtained. For example, the treatment solution may be a solution obtained by mixing the salt tolerance imparting agent with the initial growth solution, a solution obtained by mixing the salt tolerance imparting agent with the nutrient solution used in the cultivation step, or a solution having a salt composition different from those of the initial growth solution and the nutrient solution. The sodium chloride concentration of the treatment solution used in the present invention is preferably 1 % by mass or more, and more preferably the same as the sodium chloride concentration of the nutrient solution.

The salt tolerance imparting agent used in the present invention may be a drug, a microorganism, or a culture supernatant of the microorganism. Examples of the drug include pyrroloquinoline quinone (see Japanese Patent No. 5013326) and strigolactone. Examples of the microorganism include Paenibacillus Fukuinensis (see Japanese Unexamined Patent Application, First Publication No. 2013-75881). The salt tolerance imparting agent may be formed of one kind of microorganism, or a mixture of two or more kinds of microorganisms.

The concentration of the salt tolerance imparting agent in the treatment solution can be appropriately adjusted in consideration of the kind of the salt tolerance imparting agent, the kind of the plant, the growth stage or the like. When the concentration of the salt tolerance imparting agent in the treatment solution is too low, the salt tolerance imparting agent is less likely to get in contact with the root of the plant in the treatment solution, which may result in insufficient salt tolerance imparting effect. On the other hand, depending on the kind of the salt tolerance imparting agent, the growth of the plant may be adversely affected by excessive intake of the salt tolerance imparting agent. In view of this, an appropriate concentration of the salt tolerance imparting agent in the treatment solution for obtaining sufficient salt tolerance imparting effect can be determined empirically. For example, when the salt tolerance imparting agent is the microorganism, the concentration of the microorganism in the treatment solution may be set to be 10³ CFU/mL or more, whereby sufficient salt tolerance imparting effect can be obtained. When the salt tolerance imparting agent is the microorganism, the concentration of the microorganism in the treatment solution is preferably 10⁴ CFU/mL or more, and more preferably 10⁵ CFU/mL or more. When the salt tolerance imparting agent is the microorganism, an upper limit of the concentration of the microorganism in the treatment solution is not particularly limited, but for example, the concentration may be up to 10¹³ CFU/mL, whereby the quality of the treatment solution can be favorably maintained. The upper limit of the concentration of the microorganism in the treatment solution is preferably 10¹² CFU/mL, more preferably 10¹¹ CFU/mL, still more preferably 10¹⁰ CFU/mL, particularly preferably 10⁹ CFU/mL. The range of the concentration of the microorganism in the treatment solution may be, for example, 10³ to 10¹³ CFU/mL, preferably 10⁴ to 10¹² CFU/mL, more preferably 10⁵ to 10¹¹ CFU/mL, still more preferably 10⁵ to 10¹⁰ CFU/mL, especially preferably 10⁵ to 10⁹ CFU/mL.

The time period for performing the salt tolerance imparting treatment time once, that is, the time for holding at least a part of the root of the plant in the treatment solution, can be appropriately adjusted in consideration of the kind of the plant or the kind of the salt tolerance imparting agent to be used. For example, such time period for the salt tolerance imparting treatment is preferably 1 hour or more, more preferably 18 hours or more, still preferably one day or more, and further more preferably one day to seven days. Cultivating the plant for 1 hour or more with the root thereof being held in the treatment solution ensures sufficient chances for the salt tolerance imparting agent in the treatment solution to contact the root of the plant, whereby the salt tolerance can be imparted more easily.

In the initial growth step, when the seedling is to be grown in a state of being supported by the support retained within the cultivation pot, the cultivation pot may be installed in a treatment tank containing the treatment solution such that the root growing from below the support contacts the treatment solution, thereby carrying out the salt tolerance imparting treatment. For example, the root can be brought into contact with the treatment solution by using a float which has one or more through-holes for fitting the cultivation pot thereinto and is floated on the surface of the treatment solution, and fitting the cultivation pot into the float. The cultivation pot may be detachably fitted into the through-hole of the float, or may be fixed undetachably to the through-hole of the float. Alternatively, the float and the cultivation pot may be integrally formed. As a material of the float to be floated on the surface of the treatment solution, the same material as the float to be floated on the surface of the nutrient solution described later can be used.

The larger the amount of the treatment solution used in the salt tolerance imparting treatment, the larger the amount of the salt tolerance imparting agent needed. Therefore, by reducing the amount of the treatment solution to an amount that is necessary and sufficient to allow the root of the plant grown from the bottom surface of the cultivation pot to contact the treatment solution, the amount of the salt tolerance imparting agent required for performing the salt tolerance imparting treatment once can be suppressed. However, when the amount of the treatment solution is too small, it may become impossible to allow a sufficient amount of the salt tolerance imparting agent to contact the root of the plant. Therefore, when a plate into which one cultivation pot is fitted is installed per treatment tank, the amount of the treatment solution contained in the treatment tank is preferably at least 5 mL.

Thereafter, the seedling to which the salt tolerance is imparted by the salt tolerance imparting step is grown by hydroponics with the nutrient solution having a sodium chloride concentration of 1 % by mass or more.

In the present invention, the sodium chloride concentration of the nutrient solution in the cultivation step is not limited as long as the sodium chloride concentration is 1 % by mass or more, and may be appropriately adjusted in accordance with the salt tolerance of the plant to be cultivated. The sodium chloride concentration of the nutrient solution is preferably 1 to 4 % by mass, more preferably 1.5 to 3.8 % by mass, further preferably 2 to 3.5 % by mass, and particularly preferably 2.5 to 3.3 % by mass.

The nutrient solution used in the present invention preferably contains magnesium chloride in addition to sodium chloride, where the amount of magnesium chloride contained is preferably 0.5 % by mass or less, more preferably 0.1 to 0.5 % by mass.

In addition to sodium chloride and magnesium chloride, it is preferable that the nutrient solution used in the present invention contains various nutrient components which are necessary for the growth of the plant. The nutrient components can be appropriately adjusted according to the type of the plant to be cultivated. Especially, it is preferable that the nutrient solution contains elements necessary for the growth of the plant in the form of salts. Examples of such elements include nitrogen, phosphorus, potassium, calcium, magnesium, sulfur, iron, manganese, copper, molybdenum, and boron. The nutrient solution may further contain elements such as aluminum and silicon in the form of salts thereof, depending on the type of the plant. Further, the composition of the nutrient solution may be varied according to the growth stage of the plant.

The nutrient solution to be used in the present invention may be, for example, a solution prepared by supplementing deficient salt such as sodium chloride to commercially available liquid fertilizer or a solution obtained by diluting commercially available concentrated liquid fertilizer with sea water instead of water. Further, the nutrient solution may also be a solution obtained by appropriately adding a deficient salt such as salt of phosphorus to seawater.

In the present invention, the hydroponics in the cultivation step may be performed by a general hydroponics method, except that the sodium chloride concentration of the nutrient solution is set to 1 % by mass or more. The cultivation step may be performed by a deep flow technique in which a relatively large amount of the nutrient solution is stored in the cultivation tank, or a nutrient film technique in which a culture solution is allowed to flow down little by little on a flat surface having a gentle slope.

In the deep flow technique, the replacement of the nutrient solution in the cultivation tank may be carried out by a circulation method in which the nutrient solution used is circulated, or a non-circulation method in which the nutrient solution used for a certain period of time in the cultivation tank is discharged. In case of the circulation method, the nutrient solution prepared in a nutrient solution preparation tank is charged into the cultivation tank by a pump or the like, and is collected back to the nutrient solution preparation tank from the cultivation tank, and the nutrient component or the like is prepared.

For example, the deep flow technique can be carried out by using a hydroponic apparatus having a cultivation tank for storing the nutrient solution, a cultivation pot for accommodating the plant, one or more through-holes for fitting the cultivation pot, and a float to be floated on the surface of the nutrient solution. The cultivation pot may be detachably fitted into the through-hole of the float, or may be fixed undetachably to the through-hole of the float. Alternatively, the float and the cultivation pot may be integrally formed. The cultivation tank may be installed indoors, or may be installed outdoors.

In the case of a circulation type hydroponic apparatus, the cultivation tank includes a water supply hole for injecting the nutrient solution, and a drainage hole for draining the nutrient solution. In the case of a non-circulation type hydroponic apparatus, the cultivation tank may include both of the water supply hole and the drainage hole, or may include a water supply/drainage hole used for both of the water supply and the drainage. The water supply and drainage of the nutrient solution in the cultivation tank are controlled by a pump and a valve.

The cultivation pot is a container that has opening portions at least on an upper surface and a lower surface, and is capable of retaining the support. In general, the cultivation pot formed of a resin material such as polyethylene, polypropylene, or polyvinylidene chloride is used. As the support to be retained in the cultivation pot, any of those described above can be used.

The float is formed of a material that floats on the surface of the nutrient solution in a state where the cultivation pot being used for cultivating the plant is fitted into the through-hole. As such a material, for example, a foamed resin such as polystyrene foam or polypropylene foam is used. By fitting the cultivation pot into the float, the cultivation pot can be constantly positioned on the surface of the nutrient solution, regardless of a large or small amount of the nutrient solution, and even if the amount of the nutrient solution is small, the root of the plant can be allowed to contact constantly with the nutrient solution.

The float to be floated in the cultivation tank may be one sheet, or two or more sheets. When the cultivation tank is installed outdoors, it is preferable that the float is installed to cover most of the surface of the nutrient solution, in order to prevent evaporation of the nutrient solution from the surface thereof.

In the deep flow technique, it is preferable that the hydroponic apparatus used includes oxygen supply means for keeping the dissolved oxygen content of the nutrient solution at a predetermined level or higher. As the oxygen supply means, for example, an air pump or an air sucker can be used. By installing the air pump in the cultivation tank, air including oxygen can be directly supplied to the nutrient solution in the cultivation tank. When the air sucker is used, the nutrient solution can be charged into the cultivation tank after the nutrient solution is mixed with air by passing the nutrient solution through the air sucker or the like in advance.

The pH suitable for the hydroponics varies depending on the kind of the plant, and is generally approximately 5.5 to 6.5, but the pH of the nutrient solution tends to increase as the cultivation period extends longer. Therefore, in order to stably perform the hydroponics for a long period of time, it is preferable that the hydroponic apparatus used includes pH control means for measuring the pH of the nutrient solution periodically, and dosing an acid material in order to adjust the pH within a predetermined range as necessary. As the acid material used for the pH adjustment, for example, hydrochloric acid, sulfuric acid, or nitric acid can be used.

In the tomato production method of the present invention, the salt tolerance imparting step and the cultivation step may be performed in the same cultivation tank, or the salt tolerance imparting step may performed in the treatment tank storing the treatment solution, followed by transferring the seedling after the treatment to the cultivation tank storing the nutrient solution.

In the initial growth step, when the salt tolerance imparting treatment is performed in the treatment tank after the seedling is grown in a state of being supported by the support retained within the cultivation pot, the cultivation pot with the seedling may be detached from the float of the treatment tank, and may be fitted into the through-hole of the float floating on the surface of the nutrient solution stored in the cultivation tank, or the float into which the cultivation pot is embedded may be transferred from the treatment tank to be floated on the surface of the nutrient solution in the cultivation tank. Transfer means for transferring the cultivation pot or the float to the cultivation tank from the treatment tank is not particularly limited, and for example, the transfer may be performed by means of a water current, or a conveyor. When a plurality of cultivation pots are installed per treatment tank, it is preferable that a bubbling treatment is performed by the air pump, in order to prevent stagnation of the treatment solution, and oxygen deficiency.

When the salt tolerance imparting treatment is performed in the cultivation tank, first, the treatment solution is charged to the cultivation tank, and the root grown downward in the cultivation pot fitted into the float is brought into contact with the treatment solution to thereby perform the salt tolerance imparting treatment. In order to prevent the occurrence of concentration gradient of the salt tolerance imparting agent, it is preferable that the treatment solution is contacted with the root of the plant while suppressing the amount of the water supply and drainage or without the water supply and drainage.

However, when the amount of the water supply and drainage is small or the water supply and drainage is not performed, the stagnation may occur in the cultivation tank, causing adverse effect on the plant itself. Therefore, it is preferable that the treatment solution is suitably stirred by the bubbling treatment with the air pump.

After the salt tolerance imparting treatment, the treatment solution in the cultivation tank is drained, and subsequently, the nutrient solution prepared in advance in another tank is supplied to the cultivation tank. Then, the cultivation step is initiated by supplying water and draining under normal conditions. When the salt tolerance imparting agent is composed of a material, such as the microorganism, which does not adversely affect the plant even in the event of excessive intake of the material, the nutrient solution may be supplied without draining the treatment solution, and the water supply and drainage may be initiated under the normal water supply and drainage conditions.

The cultivation with the nutrient solution having a sodium chloride concentration of 1 % by mass or more (hereinafter, also referred to as "under the high salt concentration environment") need not necessarily be performed throughout the entire cultivation period after the salt tolerance imparting treatment. For example, the cultivation under the high salt concentration environment may be performed only for an arbitrary period after the salt tolerance imparting treatment. In this case, it is preferable that the cultivation under the high salt concentration environment is performed for a predetermined period immediately after the salt tolerance imparting treatment. It is speculated that, by performing the cultivation under the high salt concentration environment at an arbitrary time immediately after the salt tolerance imparting treatment, the salt tolerance imparted by the salt tolerance imparting treatment is maintained, whereby the amino acid contents of the tomato improve. The period of the cultivation under the high salt concentration environment is not particularly limited, but for example, may be a period of approximately 1/3 of the entire cultivation period, a period of approximately 1/2 of the entire cultivation period, or a period of approximately 2/3 of the entire cultivation period, from immediately after the salt tolerance imparting treatment. From the viewpoint of improving the amino acid contents of the tomato, it is preferable that the cultivation under the high salt concentration environment is performed throughout the entire cultivation period, from immediately after the salt tolerance imparting treatment.

If the seedling with only insufficient salt tolerance imparted is cultivated under the high salt concentration environment for a certain period in the cultivation step, the seedling withers. The withered plant causes the rotting, and allows undesirable bacteria or the like to proliferate in the nutrient solution. In such a case, despite the efforts made to impart the salt tolerance to the seedling, the seedling may wither by the disease or the like, due to contamination of the nutrient solution. Therefore, it is preferable that a removal step of removing the withered seedling is performed after the salt tolerance imparting step or during the cultivation step. Especially when the salt tolerance imparting step is performed by using the treatment solution having a sodium chloride concentration of 1 % by mass or more, it is preferable that the removal step is performed after the salt tolerance imparting step and before initiation of the cultivation step. In the cultivation of crops, the yield in an actual cultivation area can be improved by removing the withered seedling from the cultivation tank.

When plants are grown for a certain period under the high salt concentration environment after initiation of the salt tolerance imparting treatment, the seedling which has grown without withering can be confirmed to be a plant having the salt tolerance thereof surely improved by the salt tolerance imparting agent. By removing the withered seedling, the salt tolerant seedling produced according to the present invention can acquire quality assurance as a salt tolerant seedling.

The cultivation step may be performed indoors using the cultivation tank installed indoors, or may be performed outdoors in the open air using the cultivation tank installed outdoors.

The fruits of the tomatoes are harvested from the tomatoes cultivated until the harvest stage of the fruits. The tomato production method of the present invention may further include a selection step of selecting, from the harvested fruits of the tomato, those satisfying the above-mentioned respective ranges with respect to the amounts of free amino acids, the sodium content, water content, or sugar content of the edible part of the fruit of the tomato.

According to the tomato production method of the present invention, the tomato can be produced by using salt water or sea water.

### Examples

Hereinbelow, the present invention will be described with reference to Examples which, however, should not be construed as limiting the present invention.

### [Example 1A]

After the surfaces of seeds of a tomato (Kanpuku) were sterilized with hypochlorous acid, the seeds were sowed on a sponge sufficiently including water (freshwater). After budding, seedlings were grown for two weeks, and were grown for one week in the natural environment while gradually lowering humidity in order to acclimatize the seedlings to the environment. The seedlings were planted in a hydroponic bed, and were further grown for several days.

Microorganisms described in Plant, Cell and Environment, (2009) 32, 1682-1694 were incubated and centrifuged, thereby obtaining a pelletized microorganisms.

The freshwater in a water tank of the hydroponic bed described above was replaced with salt water (sodium chloride concentration of 1 % by mass). A suspension obtained by resuspending the pelletized microorganisms obtained above in a buffer solution was added to the salt water in the water tank, thereby preparing a treatment solution. Using the prepared treatment solution, a salt tolerance imparting treatment was performed by allowing a root of the plant to stay in contact with the microorganisms for 3 hours or more.

Next, the treatment solution of the water tank of the hydroponic bed was replaced with sea water (sodium chloride concentration of 1 % by mass), and hydroponics was performed in a greenhouse. In the salt water used in this Example, various nutrients necessary for the hydroponics were added.

Borne fruits were harvested from the plant cultivated above, and the amount (% by mass) of sodium contained in the edible part of the fruit of tomato was measured (Examples 1A-1 to 1A-3). The results are shown in Table 1.

### [Comparative Example 1A]

A fruit of tomato marketed under the trade name "Shio tomato" (Salt tomato) was obtained, and the amount (% by mass) of sodium contained in the edible part of the fruit of tomato was measured (Comparative Examples 1A-1 to 1A-4). The results are shown in Table 1.

The amount (% by mass) of sodium contained in the edible part of the fruit of tomato, which is described in the Standard Tables of Food Composition in Japan 2015 (Seventh Revised Version), is also shown in Table 1.

### [Comparative Example 2A]

After the surfaces of seeds of a tomato (Kanpuku) were sterilized with hypochlorous acid, the seeds were sowed on a sponge sufficiently including water (freshwater). After budding, seedlings were grown for two weeks, and were grown for one week in the natural environment while gradually lowering humidity in order to acclimatize the seedlings to the environment. The seedlings were planted in the hydroponic cultivation bed, and the hydroponic cultivation was performed in the greenhouse. In the hydroponics, a hydroponic solution including the nutrient for the hydroponics was used.

**[Table 1]**

| | Example 1A | | | Comparative Example 1A | | | | Standard Tables of Food Composition in Japan |
|---|---|---|---|---|---|---|---|---|
| | 1A-1 | 1A -2 | 1A-3 | 1A-1 | 1A -2 | 1A-3 | 1A-4 | |
| Sodium [% by mass] | 0.22 | 0.28 | 0.21 | 0.06 | 0.09 | 0.039 | 0.104 | 0.003 |
| Water [% by mass] | - | 87.2 | - | - | - | - | - | 94.0 |

| | | | | | | | | |
|---|---|---|---|---|---|---|---|---|
| 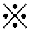 "-" in the table means "not unmeasured". | | | | | | | | |

The results of Example 1A revealed that the fruits of the tomatoes obtained in Example 1A had higher sodium concentration and lower water content than the fruit of tomato described in the Standard Tables of Food Composition in Japan and the fruits of the tomatoes obtained in Comparative Example 1A.

Separately from the tomatoes used above for measurements of sodium content and the water content, a tomato was obtained by the same cultivation method as in Example 1A except that the hydroponics was carried out using sea water (sodium chloride concentration : 3 % by mass), and the sugar content (Brix) of the edible portion of a fruit of the tomato was measured. As a result, the sugar content (Brix) of the edible portion of the fruit was found to be 12.9. In the Examples and Comparative Examples, the sugar content (Brix) was measured with respect to fruit juices of tomatoes using a sugar content refractometer.

### (Shelf Life)

After harvesting the tomato fruits of Example 1A and Comparative Example 2A, the fruits were allowed to stand at room temperature, and the degree of damage (degree of putrefaction) of the fruits with the lapse of time was observed. As a result, it was found that the fruits of the tomatoes obtained in Example 1A were less likely to rot and had superior shelf life, as compared to those obtained in Comparative Example 2A.

### [Example 2A]

A tomato (Kanpuku) was grown by hydroponics by the same cultivation method as in Example 1A, and the resulting fruits borne on the tomato plant were harvested.

After harvesting the tomato fruits, the fruits were allowed to stand at room temperature, and the degree of damage (degree of putrefaction) of the fruits with the lapse of time was observed (Examples 2A-1 and 2A-2). As the nutrient solution for hydroponics, salt water (sodium chloride concentration : 1 % by mass (Example 2A-1)) or sea water (sodium chloride concentration : 3 % by mass) (Example 2A-2)) was used. To the nutrient solution, various nutrients necessary for the hydroponics were added. The results are shown in Table 2.

### [Comparative Example 3A]

A tomato (Kanpuku) was grown by hydroponics by the same cultivation method as in Comparative Example 2A, and the resulting fruits borne on the tomato plant were harvested. After harvesting the tomato fruits, the fruits were allowed to stand at room temperature, and the degree of damage (degree of putrefaction) of the fruits with the lapse of time was observed (Comparative Example 3A-1). The results are shown in Table 2.

**[Table 2]**

| | Example 2A | | Comparative Example 3A |
|---|---|---|---|
| | 2A-1 | 2A-2 | 3A-1 |
| Nutrient solution | Salt water (NaCl 1% by mass) | Seawater (NaCl 3% by mass) | Freshwater |
| Day 1 after harvesting | Normal | Normal | Normal |
| Day 6 after harvesting | Normal | Normal | Start to deform |
| Day 14 after harvesting | Over-riped | Over-riped | Start to decay |
| Day 20 after harvesting | Over-riped | Over-riped | Decay |

The tomato fruits obtained in Comparative Example 3A began to deform from day 6 after harvesting, and began to decay on day 14 after harvesting. On day 20 after harvesting, the decay had progressed and leakage of fruit juice was observed.

On the other hand, regarding the tomato fruits obtained in Example 2A, both of the fruits obtained in Examples 2-1 and 2-2 had reached a state of being over-riped on day 14 after harvesting, but did not begin to decay even on day 20 after harvesting.

### [Example 1B]

After the surfaces of seeds of a tomato (Kanpuku) were sterilized with hypochlorous acid, the seeds were sowed on a sponge sufficiently including water (freshwater). After budding, seedlings were grown for two weeks, and were grown for one week in the natural environment while gradually lowering humidity in order to acclimatize the seedlings to the environment. The seedlings were planted in a hydroponic bed, and were further grown for several days.

Microorganisms described in Plant, Cell and Environment, (2009) 32, 1682-1694 were incubated and centrifuged, thereby obtaining a pelletized microorganisms.

The freshwater in a water tank of the hydroponic bed described above was replaced with salt water (sodium chloride concentration of 1 % by mass). A suspension obtained by resuspending the pelletized microorganisms obtained above in a buffer solution was added to the salt water in the water tank, thereby preparing a treatment solution. Using the prepared treatment solution, a salt tolerance imparting treatment was performed by allowing a root of the plant to stay in contact with the microorganisms for 3 hours or more.

Next, the treatment solution of the water tank of the hydroponic bed was replaced with salt water (sodium chloride concentration of 1 % by mass), and hydroponics was performed in a greenhouse. In the salt water or sea water used in this Example, various nutrients necessary for the hydroponics were added.

### [Comparative Example 1B]

After the surfaces of seeds of a tomato (Kanpuku) were sterilized with hypochlorous acid, the seeds were sowed on a sponge sufficiently including water (freshwater). After budding, seedlings were grown for two weeks, and were grown for one week in the natural environment while gradually lowering humidity in order to acclimatize the seedlings to the environment. The seedlings were planted in the hydroponic bed, and the hydroponics was performed in the greenhouse. In the hydroponics, a hydroponic solution including the nutrient for the hydroponics was used.

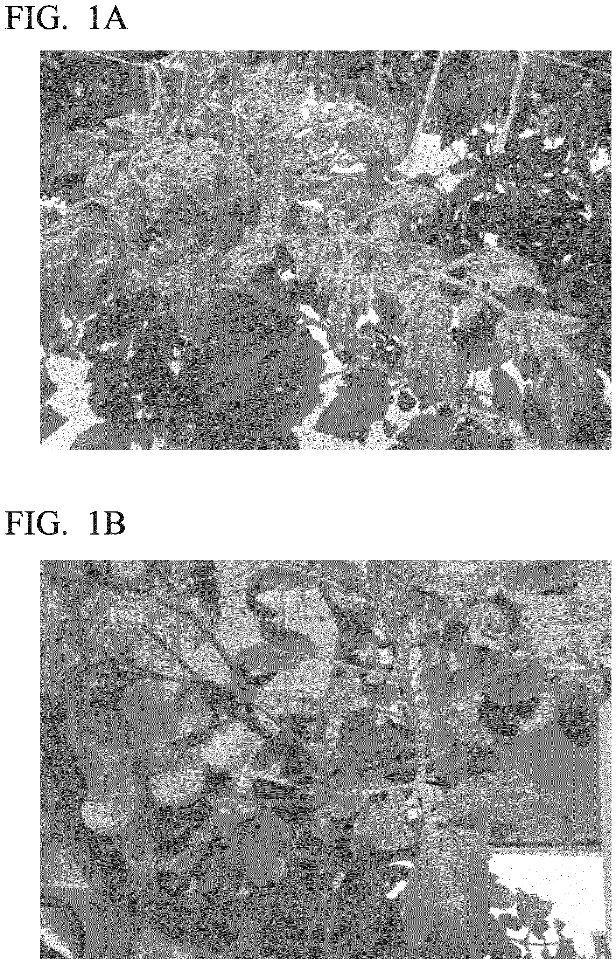

FIG. 1A is a photograph of the tomatoes grown by hydroponics in Comparative Example 1B. FIG. 1B is a photograph of the tomatoes grown by hydroponics in Example 1B.

In the tomato plants cultivated in Comparative Example 1B, plants with leaves having yellow spots in the vicinity of the respective growing points were observed, and five plants out of ten plants were confirmed to have been infected with the tomato yellow leaf curl virus (TYLCV).

On the other hand, in the tomato plants cultivated in Example 1B, none of the ten cultivated plants were confirmed to have been infected with TYLCV.

The tomato yellow leaf curl disease is a viral disease in which the tomato yellow leaf curl virus (TYLCV) is a cause of the disease. It is considered that the tomato yellow leaf curl virus is transmitted by the medium of a whitefly, and has strong spreading power. In Example 1B and Comparative Example 1B, the tomatoes were cultivated simultaneously in the same greenhouse; however, in the tomato cultivated in Example 1B, the infection with the yellow leaf curl virus was not recognized. The above results indicate that the tolerance against viral disease was imparted to the tomato by the tolerance imparting method of the present invention.

### [Example 2B]

A tomato (Kanpuku) was grown by hydroponics by the same cultivation method as in Example 1B. As the nutrient solution for hydroponics, salt water (sodium chloride concentration : 3 % by mass) was used. To the nutrient solution, various nutrients necessary for the hydroponics were added.

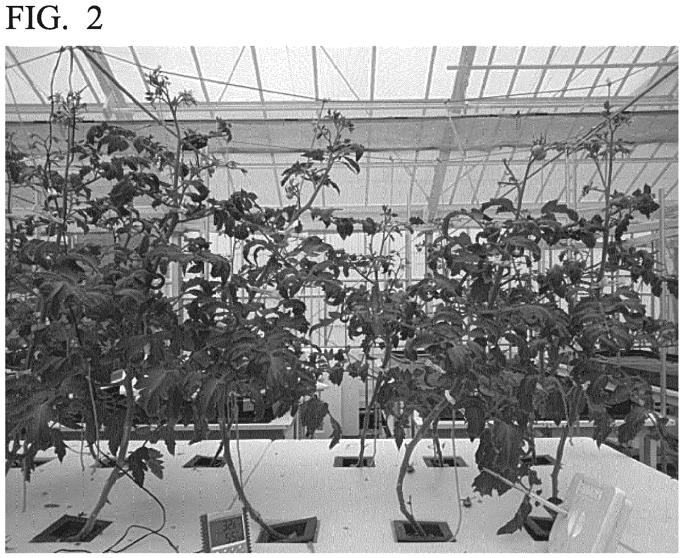

FIG. 2 is a photograph of the tomatoes grown by hydroponics in Example 2B.

None of the tomato plants cultivated in Example 1B were confirmed to have been infected with Tomato yellow leaf curl virus.

### [Example 3B]

A tomato (Kanpuku) was grown by hydroponics by the same cultivation method as in Example 1B. As the nutrient solution for hydroponics, salt water (sodium chloride concentration : 1.5 % by mass) was used. To the nutrient solution, various nutrients necessary for the hydroponics were added.

### [Comparative Example 2B]

A tomato (Kanpuku) was grown by hydroponics by the same cultivation method as in Comparative Example 1B.

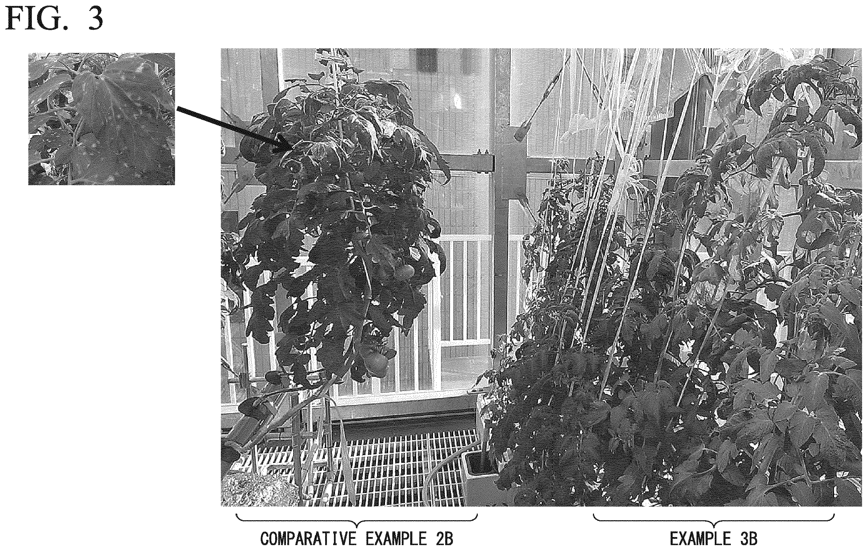

FIG. 3 shows photographs of the tomatoes grown by hydroponics in Example 3B and Comparative Example 2B. In the tomato plants cultivated in Comparative Example 2B, eight plants out of eight plants were confirmed to have been infected with powdery mildew.

On the other hand, in the tomato plants cultivated in Example 3B, none of the 24 cultivated plants were confirmed to have been infected with powdery mildew.

Powdery mildew is a fungal plant disease in which the pathogen is an ascomycete belonging to the Erysiphaceae family. Powdery mildew infections are airborne; therefore, it is generally considered that infections will spread through the population of plants grown in the same room. However, in spite of the fact that the tomato plants were cultivated were cultivated simultaneously in the same greenhouse in Example 3B and in Comparative Example 2B, only the tomato plants cultivated in Comparative Example 2B were infected with powdery mildew, while the tomato plants cultivated in Example 3B were not. This result shows that by the tolerance imparting method of the present invention, the tolerance against powdery mildew was imparted to the tomato.

### [Example 1C]

After the surfaces of seeds of a tomato (Kanpuku) were sterilized with hypochlorous acid, the seeds were sowed on a sponge sufficiently including water (freshwater). After budding, seedlings were grown for two weeks, and were grown for one week in the natural environment while gradually lowering humidity in order to acclimatize the seedlings to the environment. The seedlings were planted in a hydroponic bed, and were further grown for several days.

Microorganisms described in Plant, Cell and Environment, (2009) 32, 1682-1694 were incubated and centrifuged, thereby obtaining a pelletized microorganisms.

The freshwater in a water tank of the hydroponic bed described above was replaced with salt water (sodium chloride concentration of 1 % by mass). A suspension obtained by resuspending the pelletized microorganisms obtained above in a buffer solution was added to the salt water or the sea water in the water tank, thereby preparing a treatment solution. Using the prepared treatment solution, a salt tolerance imparting treatment was performed by allowing a root of the plant to stay in contact with the microorganisms for 3 hours or more.

Next, the treatment solution of the water tank of the hydroponic bed was replaced with sea water (sodium chloride concentration of 3 % by mass), and the hydroponics was performed in the greenhouse. In the salt water or sea water used in this Example, various nutrients necessary for the hydroponics were added.

A tomato fruit was harvested from the tomato plant cultivated in Example 1C, and the amounts of free amino acids and the sugar content were measured with respect to the edible portion of the tomato fruit. The results are shown in Table 3.

The amounts of free amino acids contained in the edible part of the fruit of tomato, which are described in a table for amino acids in the Standard Tables of Food Composition in Japan 2015 (Seventh Revised Version), are also shown in Table 1.

**[Table 3]**

| | | Tomato fruit (Example 1C - seawater cultivation) | Tomato fruit (Standard Tables of Food Composition in Japan) |
|---|---|---|---|
| | Arginine | 23 | 3.2 |
| | Lysine | 21 | 5.9 |
| | Histidine | 26 | - |
| | Phenylalanine | 27 | 10.6 |
| | Tyrosine | 10 | 3.5 |
| | Leucine | 8 | 3.7 |
| | Isoleucine | 18 | 3.7 |
| Free amino acid [mg / 100 g] | Methionine | 4 | - |
| | Valine | 8 | 3.2 |
| | Alanine | 55 | 4.1 |
| | Glycine | 9 | 1.1 |
| | Proline | 26 | 106 |
| | Glutamic acid | 573 | 93.6 |
| | Serine | 104 | 7.8 |
| | Threonine | 26 | 5.5 |
| | Aspartic acid | 122 | 22.7 |
| | Tryptophan | 4 | - |
| | Cysteine | <1 | - |
| Sugar content (Brix) | | 12.9 | - |

The results of Example 1C revealed that the fruit of the tomato obtained in Example 1C contained free amino acids in larger amounts per gram than the fruit of typical tomato described in the Standard Tables of Food Composition in Japan. Especially, in Example 1C, the amounts per gram of free amino acids such as arginine, isoleucine, alanine, glutamic acid, serine and aspartic acid were 5 times or more higher than those of typical tomato fruit shown in the Standard Tables of Food Composition in Japan. Especially, the amounts of glutamic acid known as flavor ingredient and aspartic acid were large.

Separately from the tomatoes used above for measurements of the amounts of free amino acids and the sugar content, tomatoes were obtained by the same cultivation method as in Example 1C except that the hydroponics was carried out using salt water (sodium chloride concentration : 1 % by mass), and the sodium contents and water contents of the edible portions of the tomatoes were measured. As a result, the sodium contents of the edible portions of three tomato fruits were found to be 0.22 % by mass, 0.28 % by mass and 0.21 % by mass, and the water content of one tomato fruit was found to be 87.2 % by mass.

### [Example 2C]

A tomato (Kanpuku) was grown by hydroponics by the same cultivation method as in Example 1C, and the resulting fruits borne on the tomato plant were harvested. As the nutrient solution for hydroponics, salt water (sodium chloride concentration : 1 % by mass (Example 2C-1) or sodium chloride concentration : 2 % by mass (Example 2C-2)) or sea water (sodium chloride concentration : 3 % by mass) (Example 2C-3)) was used. To the nutrient solution, various nutrients necessary for the hydroponics were added.

With respect to the edible parts of the obtained tomato fruits, the amounts of free amino acids and the water contents were measured (Examples 2C-1 to 2C-3). The results are shown in Table 4 and Table 5.

### [Comparative Example 1C]

After the surfaces of seeds of a tomato (Kanpuku) were sterilized with hypochlorous acid, the seeds were sowed on a sponge sufficiently including water (freshwater). After budding, seedlings were grown for two weeks, and were grown for one week in the natural environment while gradually lowering humidity in order to acclimatize the seedlings to the environment. The seedlings were planted in the hydroponic bed, and the hydroponics was performed in the greenhouse. In the hydroponics, a hydroponic solution including the nutrient for the hydroponics was used.

A borne fruit was harvested from the plant cultivated above, and the amounts of free amino acids and the sugar content were measured with respect to the edible part of the tomato fruit (Comparative Example 1C-1). The results are shown in Table 4 and Table 5.

**[Table 4]**

| | | Example 2C | | | Comparative Example 1C |
|---|---|---|---|---|---|
| | | 2C-1 | 2C-2 | 2C-3 | 1 C - 1 |
| Nutrient solution | | Salt water (NaCl 1% bv mass) | Salt water (NaCl 2% by mass) | Sea water (NaCl 3% by mass) | Fresh water |
| | Arginine | 4 | 6.3 | 12 | 5.9 |
| | Lysine | 4.4 | 7 | 10 | 3.6 |
| | Histidine | 7.6 | 12.1 | 28 | 4.6 |
| | Phenylalanine | 6.4 | 5.9 | 14 | 5.9 |
| | Tyrosine | 1.7 | 1.6 | 4 | 1.8 |
| | Leucine | 3.4 | 4.1 | 6 | 3.2 |
| | Isoleucine | 6.8 | 8.3 | 14 | 2.9 |
| Standard amino acid [mg / 100 g] | Methionine | 1 | 1.3 | 2 | 1.2 |
| | Valine | 4.1 | 3.8 | 10 | 2.8 |
| | Alanine | 9.1 | 22.9 | 12 | 3.6 |
| | Glycine | 2 | 2.9 | 3 | 1.1 |
| | Proline | 33.9 | 88.3 | 134 | 1.2 |
| | Glutamic acid | 321.9 | 650.1 | 607 | 185.9 |
| | Serine | 32.3 | 66 | 72 | 8.6 |
| | Threonine | 12.3 | 18 | 29 | 5.4 |
| | Aspartic acid | 47.9 | 87.3 | 124 | 34.6 |
| | Tryptophan | 1.3 | 1.5 | 3 | 1.6 |
| | Cysteine | 0.1 | 0.3 | <1 | 0.04 |
| Total amount [mg / 100 g] | | 500.2 | 987.7 | 1084 | 273.94 |
| Sugar content (Brix) | | 10.7 | 13.4 | - | 4.0 |

**[Table 5]**

| | | Example 2C | | | Comparative Example 1C |
|---|---|---|---|---|---|
| | | 2C-1 | 2C-2 | 2C-3 | 1 C - 1 |
| Nutrient solution | | Salt water (NaCl 1% by mass) | Salt water (NaCl 2% by mass) | Sea water (NaCl 3% by mass) | Fresh water |
| Non standard Amino acid [mg / 100 g] | Phosphoserine | 1.4 | 1.9 | - | 0.8 |
| | β-Alanine | 3.6 | 4.4 | - | 0.7 |
| | γ-aminobutyric acid (GABA) | 185.8 | 173.3 | - | 36.2 |

The above results revealed that the fruits of the tomatoes obtained in Example 2C contained proteogenic amino acids in larger amounts per gram than the fruit of the tomato obtained in Comparative Example 1C.

Further, the above results also revealed that the fruits of the tomatoes obtained in Example 2C contained nonproteogenic amino acids such as phosphoserine, β-alanine and γ-amino acid in larger amounts per gram than the fruit of the tomato obtained in Comparative Example 1C. The fruit of tomato cultivated with a nutrient solution with high sodium chloride concentration tended to have a larger total amount of proteogenic amino acids and a larger amount of amino acids in general.

### INDUSTRIAL APPLICABILITY

The present invention can provide a plant with improved shelf life as compared to the conventional plant of the same species.

The tolerance imparting method of the present invention can impart tolerance to plants against diseases and pests.

A fruit of the tomato of the present invention has free amino acid contents different from those of conventional tomatoes and has unique flavor.

The tomato production method of the present invention can provide a tomato bearing a fruit having free amino acid contents different from those of conventional tomatoes and unique flavor.

## Claims

1. A plant corresponding to an item described in Standard Tables of Food Composition in Japan 2015 (Seventh Revised Version) and having a mass content of sodium which is at least 50 times a mass content of sodium of the item described in the Standard Tables of Food Composition in Japan 2015 (Seventh Revised Version), the mass content of sodium being measured per unit mass of edible portion of the plant.

2. The plant according to claim 1, which has a sodium content of 0.15 % by mass or more as measured with respect to edible portion of a fruit of the plant.

3. The plant according to claim 1 or 2, which has a water content of 90 % by mass or less as measured with respect to edible portion of a fruit of the plant.

4. The plant according to any one of claims 1 to 3, which has a sugar content (Brix) of 8 or more as measured with respect to edible portion of a fruit of the plant.

5. The plant according to any one of claims 1 to 4, which is a tomato.

6. A method for producing the plant of any one of claims 1 to 5, comprising:
a salt tolerance imparting step of performing a salt resistance imparting treatment by bringing a salt tolerance imparting agent into contact with at least a part of a root of a plant, thereby obtaining a salt tolerance imparted plant; and
a cultivation step of hydroponically cultivating the salt tolerance imparted plant obtained in the salt tolerance imparting step with a nutrient solution having a sodium chloride concentration of 1 % by mass or more.

7. A tolerance imparting method for imparting tolerance to a plant against diseases and pests, comprising:
a salt tolerance imparting step of performing a salt resistance imparting treatment by bringing a salt tolerance imparting agent into contact with at least a part of a root of a plant, thereby obtaining a salt tolerance imparted plant; and
a cultivation step of hydroponically cultivating the salt tolerance imparted plant obtained in the salt tolerance imparting step with a nutrient solution having a sodium chloride concentration of 1 % by mass or more.

8. The tolerance imparting method according to claim 7, which further comprises, as a step to be performed prior to the salt tolerance imparting step, an initial growth step of allowing a seed or a bulb of the plant to bud and root under an environment with a sodium chloride concentration of less than 1 % by mass.

9. The tolerance imparting method according to claim 8, wherein the seed or the bulb is allowed to bud or root in the initial growth step under an environment with a sodium chloride concentration of 0.5 % by mass or less.

10. The tolerance imparting method according to any one of claims 7 to 9, wherein the salt tolerance imparting treatment is a treatment of putting at least a part of the root of the plant into a treatment solution which contains the salt tolerance imparting agent, and has a sodium chloride concentration of 1 % by mass or more.

11. The tolerance imparting method according to claim 10, wherein at least a part of the root of the plant is held in the treatment solution for 1 hour or more.

12. The tolerance imparting method according to claim 10 or 11, wherein the salt tolerance imparting agent is a microorganism that imparts the salt tolerance to the plant by adhering to the root, and
the concentration of the microorganism in the treatment solution is 10³ CFU/mL or more.

13. The tolerance imparting method according to any one of claims 7 to 12, wherein the nutrient solution further contains a magnesium chloride in an amount of 0.5 % by mass or less.

14. The tolerance imparting method according to any one of claims 7 to 13, wherein the salt tolerance imparting agent comprises at least one species of the microorganism.

15. The tolerance imparting method according to any one of claims 7 to 14, wherein the plant is a plant of Solanaceae family.

16. The tolerance imparting method according to any one of claims 7 to 15, wherein the tolerance is viral disease tolerance.

17. The tolerance imparting method according to any one of claims 7 to 15, wherein the tolerance is fungal disease tolerance.

18. A tomato satisfying at least one of the following (1) to (20), which respectively represent amounts of free amino acids contained per 100 g of edible portion of a fruit of the tomato:
(1) free glutamic acid : 200 mg or more,
(2) free aspartic acid : 40 mg or more,
(3) free arginine : 6 mg or more,
(4) free isoleucine : 6 mg or more,
(5) free alanine : 8 mg or more,
(6) free serine : 15 mg or more,
(7) free lysine : 7 mg or more,
(8) free histidine : 7 mg or more,
(9) free phenylalanine : 12 mg or more,
(10) free tyrosine : 4 mg or more,
(11) free leucine : 4 mg or more,
(12) free methionine : 2 mg or more,
(13) free valine : 3.5 mg or more,
(14) free glycine : 2 mg or more,
(15) free proline : 50 mg or less,
(16) free threonine : 10 mg or more,
(17) free tryptophan : 2 mg or more,
(18) free phosphoserine : 1.2 mg or more,
(19) free β-alanine : 2 mg or more, and
(20) free γ-aminobutyric acid : 80 mg or more.

19. The tomato according to claim 18, which satisfies at least one of (1), (2), (4) to (6), (8), (13), (14) and (16) .

20. The tomato according to claim 19, which satisfies at least one of (1), (5), (6), (14) and (16).

21. The tomato according to claim 18, which satisfies at least one of (1) to (6), wherein the amounts of free amino acids contained per 100 g of edible portion of a fruit of the tomato are as follows:
(1) free glutamic acid : 500 mg or more,
(2) free aspartic acid : 100 mg or more,
(3) free arginine : 10 mg or more,
(4) free isoleucine : 10 mg or more,
(5) free alanine : 10 mg or more, and
(6) free serine : 70 mg or more.

22. The tomato according to claim 21, which satisfies at least one of (1) and (2).

23. The tomato according to any one of claims 18 to 22, which has a sodium content of 0.15 % by mass or more as measured with respect to edible portion of a fruit of the tomato.

24. The tomato according to any one of claims 18 to 23, which has a water content of 90 % by mass or less as measured with respect to edible portion of a fruit of the tomato.

25. The tomato according to any one of claims 18 to 24, which has a sugar content (Brix) of 8 or more as measured with respect to edible portion of a fruit of the tomato.

26. A method for producing the tomato of any one of claims 18 to 25, comprising:
a salt tolerance imparting step of performing a salt resistance imparting treatment by bringing a salt tolerance imparting agent into contact with at least a part of a root of a tomato, thereby obtaining a salt tolerance imparted tomato; and
a cultivation step of hydroponically cultivating the salt tolerance imparted tomato obtained in the salt tolerance imparting step with a nutrient solution having a sodium chloride concentration of 1 % by mass or more.
